# EUROPEAN PATENT SPECIFICATION

(11) **EP 4 167 753 B1**
(45) Date of publication and mention of the grant of the patent: **21.10.2026**
(21) Application number: 21733153.7
(22) Date of filing: 23.06.2021
(51) Int. Cl.: A23L 19/15, B01D 61/22, B01D 61/58, A23J 1/00, A23J 1/16, A23J 3/14, A23L 2/14, A23L 2/08, A23L 9/10, A23L 19/12, B01D 61/14, C07K 1/34, A23L 33/185, A23L 19/00, B01D 61/02

(54) **METHOD FOR SEPARATION OF POTATO PROTEINS AND INSOLUBLE FIBERS FROM PHENOLIC AND/OR GLYCOALKALOID COMPOUNDS**
VERFAHREN ZUR TRENNUNG VON KARTOFFELPROTEINEN UND UNLÖSLICHEN FASERN AUS PHENOL- UND/ODER GLYCOALKALOIDVERBINDUNGEN
PROCÉDÉ DE SÉPARATION ENTRE PROTÉINES DE POMME DE TERRE ET FIBRES INSOLUBLES ET COMPOSÉS PHÉNOLIQUES ET/OU GLYCOALCALOÏDES

(30) Priority: 23.06.2020 DK PA202070411; 27.11.2020 DK PA202070792
(43) Date of publication of application: 26.04.2023
(73) Proprietor: Coöperatie Koninklijke Cosun U.A., 4814 NE Breda (NL)
(72) Inventor: LIHME, Allan Otto Fog, 3520 Farum (DK); HANSEN, Marie Bendix, 2000 Frederiksberg (DK); LINDVED, Bodil Kjær, 3060 Espergærde (DK); JORDENS, Rick Adrianus Petrus, 6613AS Balgoij (NL)
(74) Representative: Nederlandsch Octrooibureau
(86) International application number: PCT/EP2021/067207
(87) International publication number: WO 2021/260039

(56) References cited:
- WO-A1-02/060273
- WO-A1-2019/057257
- WO-A1-2019/215153
- US-A1- 2003 077 265
- US-A1- 2019 082 715
- US-A1- 2019 274 332
- SISSEL LOKRA ET AL: "Industrial Proteins from Potato Juice. A Review", FOOD, vol. 3, no. 1, 1 January 2009 (2009-01-01), pages 88 - 95, XP055434942
- KNUT OLAV STRÃ TKVERN ET AL: "Recovery of Native Potato Protein Comparing Expanded Bed Adsorption and Ultrafiltration", FOOD AND BIOPROCESS TECHNOLOGY ; AN INTERNATIONAL JOURNAL, SPRINGER-VERLAG, NEW YORK, vol. 5, no. 5, 15 January 2011 (2011-01-15), pages 1939 - 1949, XP035067433, ISSN: 1935-5149, DOI: 10.1007/S11947-010-0494-2

## Description

### TECHNICAL FIELD OF THE INVENTION

The present invention relates to methods for isolating (a) potato protein-fiber mixtures, particularly mixtures of functional potato proteins and insoluble fibers, and (b) phenolic and/or glycoalkaloid compounds, from potato fruit juice or derivatives thereof, as defined in the appended claims.

### BACKGROUND OF THE INVENTION

Many biological fluids and extracts contain soluble proteins and soluble phenolic compounds such as phenolic acids, flavonoids and tannins. This is particularly the case for liquid plant extracts and extracts of *e.g.* yeast and algae. Many of the phenolic compounds are highly reactive and may *e.g.* be oxidized to create oligomers and polymers. Such phenolic compounds may adsorb to, and even react covalently with, proteins. The susceptibility of phenolic compounds towards adsorption and/or their covalent reaction with proteins is influenced by many physico-chemical parameters, such as the degree of oxidation, pH, temperature and time of contact.

The potato *(Solanum tuberosum* L.) is a tuber that is largely used as food or in food applications and is a source of different bioactive compounds, such as starch, dietary fibers, amino acids, minerals, vitamins, glycoalkaloid compounds and phenolic compounds.

Potato fruit juice is a complex mixture of soluble and insoluble material comprising proteins, starch, minerals, (toxic) glycoalkaloids, (insoluble) fibers and monomeric and polymeric reactive phenols.

Phenolic compounds present in potatoes are phenolic acids and flavonoids including flavonols, flavanols, and anthocyanins. Phenolic acids are the most abundant phenolic compounds in potatoes. Chlorogenic acid, which is the ester of caffeic acid and quinic acid, constitutes 90% of the phenolic compounds in potato peels and exists in the form of three main isomers, chlorogenic acid (5-O-caffeoylquinicacid), neochlorogenic acid (3-O-caffeoylquinic acid), and cryptochlorogenic acid (4-O-caffeoylquinic acid). Also, caffeic acid is quantified at 25-72 mg/100 g in potatoes by many researchers. Other phenolic acids such as ferulic acid, gallic acid, and p-coumaric acid have also been quantified in potatoes, ranging from 0-5 mg/100 g dry weight. Furthermore, syringic acid, vanillic acid, sinapic acid, and salicylic acid are present in small quantities (see H. Akyol et al., Int. J. Mol. Sci., 2016, 17, pp 835-854).

Due to oxidation of natural phenolic compounds, potato fruit juice may turn brown or black. Chemically, the phenolic compounds are oxidized into quinones, which combine into a dark polymer residue. During the oxidation process, the potato proteins may undergo reaction and partial crosslinking. This crosslinking may dramatically reduce the solubility of the potato proteins. Thus, from a technological point of view, the complexity and instability of the potato fruit juice makes the separation and isolation of native potato proteins much more complicated and economically demanding than the isolation of proteins from other types of protein solutions.

Purification of potato proteins from potato fruit juice is further complicated by the presence of glycoalkaloids, which must be removed before the potato proteins may be used in human nutrition. Glycoalkaloids are well known anti-nutritional factors. The glycosylated forms of glycoalkaloids, such as α-solanine and α-chaconine, show the highest toxicity. The aglycons, such as solanidine, have a more than 100-fold lower liver toxicity. α-Solanine and α-chaconine make up more than 95 % of the total glycoalkaloid content in potatoes. Other glycoalkaloids are for example tomatine, tomatidenol and demissidine. Glycoalkaloids have a bitter taste and negatively affect many of the physical and/or biological properties of potato proteins. For food applications, the taste threshold of glycoalkaloids is about 140-170 mg of glycoalkaloids per kg of product. This threshold strongly limits the applications of known native potato protein isolates in foods.

Several compounds, such as proteins and metabolites, comprised in potatoes are valuable and useful in many different applications, such as in nutrition, medical treatments, cosmetics and as acceptable process aids for industrial manufacture of the same. Particularly, patatin and protease inhibitor proteins and phenolic compounds in potato fruit juice or derivatives thereof (waste products of the potato starch manufacturing industry) are interesting and become even more valuable and useful in isolated or more pure form. Patatin and protease inhibitor proteins and phenolic compounds are highly nutritious and have many desirable functional characteristics which could make them useful in both food and industrial products.

The potato fruit juice or the derivatives thereof typically also contain insoluble fibers, also known as dietary fibers. Such dietary fibers may have health promoting effects and add functionality when applied as a food ingredient.

Native/soluble or at least partially native/soluble patatin and/or protease inhibitor proteins, *i.e.* not fully denatured or coagulated potato proteins, may be used as techno-functional ingredients in the preparation of foodstuffs, for example to provide (thermo)gelling, foaming, water-binding or emulsification properties. For these purposes, the proteins preferably are devoid of compounds that may give rise to unwanted color and taste formation and/or influence the quality and stability of the prepared food.

Thus, separation or isolation of (a) native/soluble potato proteins and (b) phenolic and/or glycoalkaloid compounds from potato fruit juice or a derivative thereof is important for obtaining the necessary purity and quality of these valuable compounds.

Membrane filtration, in particular cross-flow ultrafiltration and diafiltration, is a well-known method for separation of high molecular weight compounds, like proteins, from low molecular weight compounds like small carbohydrates, organic acids and minerals (salts). A widespread example of this technique is the manufacture of whey protein concentrates and whey protein isolates from cheese whey by combined ultrafiltration and diafiltration.

Diafiltration, in the context of the present invention, is a technique that uses membranes to completely remove, replace, or lower the concentration of salts and/or other low molecular weight substances from solutions containing proteins, peptides, nucleic acids, and other high molecular weight biomolecules. The process selectively utilizes permeable (porous) membrane filters to separate the components of solutions and suspensions based on their molecular size. An ultrafiltration membrane retains molecules that are larger than the pores of the membrane while smaller molecules such as salts, phenolic and/or glycoalkaloid compounds, solvents and water, may pass through the membrane. In a diafiltration process, water is added to the retentate, while the membrane filtration process continuously removes water, salts and low molecular weight compounds to the permeate side of the membrane.

B. Dutré and G. Trägårdh, Macrosolute-microsolute separation by ultrafiltration: A review of diafiltration processes and applications, Desalination, 1994, 95, pp 227-267, disclose a review of the theory and applications of diafiltration in several commercial scale examples.

The membrane processes disclosed in the prior art to remove phenolic and/or glycoalkaloid compounds from potato fruit juice typically start with a pretreatment step wherein (substantially) all of the insoluble material, including insoluble fibers, is removed prior to ultrafiltration/diafiltration.

WO97/42834A1, for example, describes in Example 1 a process for the production of undenatured potato protein from potato fruit juice. The potato fruit juice was pretreated causing flocculation of contaminations and subjected to disc stack centrifuging to remove flocculates, other solids and microorganisms. The disc stack centrifugation necessarily removes substantially all of the insoluble fibers present in the potato fruit juice. The supernatant obtained after centrifugation was concentrated about 12 times by ultrafiltration using a continuous ultrafiltration unit equipped with polyethersulfone membranes, cut-off value 5 kDa. The retentate was washed by means of diafiltration in the presence of bisulphite to minimise polyphenol oxidation. Diafiltration was continued until salts and metal concentrations had reached acceptable levels. The concentrate was freeze-dried until a dry solid content of 92% was reached. The freeze-dried powder contained 70% protein and no detectable levels of glycoalkaloids. WO97/42834A1 does not comment on the removal of polyphenols from the product.

H.J. Zwijnenberg et al., Desalination, 144, 2002, pp 331-334, also disclose examples wherein native protein is recovered from potato fruit juice by ultrafiltration and subsequent ultrafiltration. It is described in Zwijnenberg that the potato fruit juice applied contains everything except the starch and fiber. A pretreatment was performed prior to ultrafiltration to reduce the amount of fibers. Diafiltration with tap water was carried out in order to wash out low molecular substances like amino-acids, phenolic components, glycoalkaloids and potassium. The resulting protein was spray-dried in order to get a stable and dry product that could be transported and stored during longer periods.

WO2019/215153A1 discloses a process for separation of one or more potato proteins from a group of impurities, amongst which is phenols, in an aqueous solution, said process comprising the steps of:
a) providing an aqueous solution comprising the one or more potato proteins and a group of impurities;
b) optionally subjecting the aqueous solution to a pretreatment to clarify and remove suspended non-soluble matter;
c) precipitating the one or more potato proteins to create a suspension of precipitated potato protein in the solution containing impurities;
d) subjecting the suspension to a membrane filtration process wherein at least one member of the group of impurities passes the membrane as a permeate and the one or more precipitated potato proteins are concentrated in the retentate;
e) optionally diafiltering the retentate with one or more solvents to further remove impurities into the permeate;
f) optionally re-solubilizing the precipitated one or more potato proteins; and
g) optionally separating the group of impurities in said permeate into at least two individual fractions.

In the working examples of WO2019/215153A1, fibers and other insoluble material are removed by centrifugation.

US2003/077265A1 concerns an ultrafiltration method for the isolation and purification of a proteinase inhibitor extract from potato tubers. The extraction and isolation of the proteinase inhibitor from potatoes begins with the addition of an organic acid, such as formic acid, and a salt to raw potatoes. Undesired proteins are denatured using heat treatment and removed, resulting in an extract solution suitable for ultrafiltration. The ultrafiltration process comprises concentration and diafiltration.

For certain separation tasks, such as the separation of proteins from phenolic and/or glycoalkaloid compounds, and in particular the separation of soluble proteins from phenolic and/or glycoalkaloid compounds in plant extracts, the membrane filtration technology is not a complete and cost-efficient process, which may be due to the tendency of phenolic and/or glycoalkaloid compounds to adhere to other compounds such as proteins, polysaccharides as well as the membrane surface.

For example, according to several scientific reports (see *e.g.* K.O Straetkvern and J.G. Schwarz, Recovery of Native Potato Protein Comparing Expanded Bed Adsorption and Ultrafiltration, Food and Bioprocess. Technol., 5(5), 2012, pp 1939-1949 and H.J. Zwijnenberg et al., Desalination, 144, 2002, pp 331-334), ultrafiltration has low selectivity and only poorly separates polyphenols and brown polyphenol complexes from proteins, thus giving a powder with a final brown hue and higher content of chlorogenic acids. Moreover, membrane concentration of potato fruit juice often leads to fouling of the membranes with corresponding low flux rates, low system productivity and a short membrane lifetime. Both disclosures describe the use of water for the applied diafiltration step. In Zwijnenberg *et al.* it is illustrated how the permeate flux decreases from the onset of diafiltration using water as the diafiltration solvent.

US2019/274332 discloses a method for reducing glycoalkaloid content and turbidity of an aqueous phase comprising two or more of PA, PI, PPO, LipO, pectin, lipid, glycoalkaoid and phenolic compounds, wherein the aqueous phase is treated with a soluble silicate at a pH of 3-10, allowing precipitation of the silicate with one or more of PA, PI, PPO, LipO, pectin, lipid, glycoalkaoid and phenolic compounds, such that the concentration of solanine is reduced by at least 15% and the optical density at 620nm of the remaining aqueous phase is less than 0.7.

US2019/082715 discloses a method for treating root- or tuber juice, comprising
a) a pretreatment of the root- or tuber juice to remove lipids to a level below 28g/kg dry weight;
b) cooling the root- or tuber juice to a temperature of -0.3°C to -16°C to form ice crystals; and
c) separating the ice crystals from the root- or tuber juice to obtain a concentrated root- or tuber juice.

As explained hereinbefore, potato fruit juice or derivatives thereof typically contain insoluble fibers. In the prior art, these fibers are removed from the potato fruit juice before isolating the proteins. Accordingly, expensive pretreatment steps are needed and valuable ingredients are removed from the potato protein product.

It is therefore an object of the invention to provide improved methods for the separation of:
(a) native/soluble potato proteins; and
(b) phenolic and/or glycoalkaloid compounds,
from potato fruit juice or derivatives thereof at industrial scale.

It is another object of the invention to provide methods for the separation of:
(a) a mixture of native/soluble potato proteins and insoluble fibers; and
(b) phenolic and/or glycoalkaloid compounds,
from potato fruit juice or derivatives thereof at industrial scale.

It is another object of the invention to provide a simplified process for the isolation of potato protein products for food applications from potato fruit juice or derivatives thereof at industrial scale, wherein valuable ingredients, such as insoluble fibers, are preserved in the potato protein products during the whole process.

It is yet another object of the invention to provide valuable phenolic and/or glycoalkaloid compounds with sufficient purity and quality at industrial scale.

### SUMMARY OF THE INVENTION

The present inventors have unexpectedly found that one or more of the objectives can be met by subjecting potato fruit juice or derivatives thereof comprising insoluble fibers to a membrane filtration process at industrial scale using diafiltration solvents with an increased ionic strength, which leads to a higher permeability of the membrane for the phenolic and/or glycoalkaloid compounds and a higher average permeate flux during the diafiltration step. Using this process,
(a) products enriched in phenolic and/or glycoalkaloid compounds with sufficient quality; and
(b) food-grade mixtures of functional potato proteins and insoluble fibers, depleted in phenolic compounds, glycoalkaloid compounds and salts,
can be obtained at industrial scale. No expensive pretreatment steps are needed to remove insoluble fibers from the potato fruit juice or the derivatives thereof.

Accordingly, in a first aspect, the present invention provides a method for separation of (a) potato proteins and insoluble fibers from (b) first salts and phenolic and/or glycoalkaloid compounds in potato fruit juice or a derivative thereof, said method comprising the steps of:
(i) providing a potato fruit juice or a derivative thereof, comprising:
   - potato proteins; and
   - insoluble fibers; and
   - one or more first salts; and
   - phenolic and/or glycoalkaloid compounds,
   wherein the potato fruit juice or the derivative thereof has an absorbance at 600 nm in the range of 0.1 to 50, as determined using the method described in the detailed description, and contains insoluble fibers at a concentration in the range of 0.1 to 30 mg/ml, based on the dry weight of the insoluble fiber;
(ii) subjecting the potato fruit juice or the derivative thereof provided in step (i) to a first cross-flow membrane filtration process wherein water and at least a portion of the first salts and at least a portion of the phenolic and/or glycoalkaloid compounds migrate across the membrane into a first permeate and wherein the potato proteins are retained in a first retentate, said first retentate having a true protein concentration in the range of 5 g/L to 200 g/L;
(iii) adding aqueous diafiltration liquid containing one or more salts to the first retentate obtained in step (ii) to form a diluted first retentate having a conductivity of between 0.1 mS/cm and 50 mS/cm and an absorbance at 600 nm of between 0.01 and 50 and subjecting said diluted first retentate to a second cross-flow membrane filtration as diafiltration, to create a second permeate being a diafiltrate containing at least a portion of said phenolic and/or glycoalkaloid compounds and salts and a second retentate comprising potato proteins, salts and insoluble fibers. The invention is defined by the appended set of claims.

In a second aspect (not claimed), a potato fruit juice product. comprising protein and insoluble fibers obtainable via the process as defined herein is disclosed.

In a third aspect (not claimed), the invention provides a potato fruit juice product, comprising potato protein and insoluble fibers, having:
- a true potato protein content of at least 60%, such as at least 65 %, at least 70 %, at least 75 %, at least 80 %, at least 85 %, or at least 90 %;
- a content of insoluble fibers in the range of 2 to 39 %, such as in the range of 5 to 30 %, 7 to 25 %, 5 to 12 % 5 to 10 %, 5 to 8 %, 2-5%, or 3-8 % on a dry weight basis;
- an amount of phenolic compounds of less than 5000 mg phenolic compounds per kg potato protein on the basis of dry weight, such as less than 4000 mg/kg, less than 3000 mg/kg, less than 2000 mg/kg, less than 1500 mg/kg, less than 1250 mg/kg, less than 1000 mg/kg, less than 750 mg/kg, less than 500 mg/kg, or less than 200 mg phenolic compounds/kg potato protein on the basis of dry weight; and
- an amount of total glycoalkaloid compounds of less than 500 mg/kg, such as less than 300 mg/kg, such as less than 200 mg/kg, such as less than 100 mg/kg, such as less than 50 mg/kg, measured as mg total glycoalkaloid compounds/kg potato protein on the basis of dry weight.

### DEFINITIONS

The term '*potato fruit juice or derivatives thereof*' means the liquid stream after the (industrial scale) separation of potato starch from potatoes *(solanum tuberosum* L.) and any aqueous streams derived from processing of such liquid stream by for example flocculation of contaminants and centrifugation, provided that the *'derivatives thereof*' still comprise:
- protein; and
- insoluble fibers; and
- salts; and
- phenolic and/or glycoalkaloid compounds.

The term further includes any aqueous stream that may be obtained by disintegration, e.g. by grinding, shredding and/or pressing, of the raw potatoes, peels and cuts from raw potatoes with or without extraction by addition of water or an aqueous extraction solution in combination with physical disruption of the plant tissue and/or cells. The potato fruit juice may also be obtained from potatoes, peels and cuts that have been subjected to a heating process and/or have been subjected to freezing.

The term '*potato protein(s)'* means protein or proteins present in the tubers of the potato plant *solanum tuberosum* L., particularly patatin and/or protease inhibitor.

The term '*functional proteins'* means proteins that (still) have a high level of their intrinsic techno-functional properties, such as aqueous solubility, the ability to form gels when heated in solution (thermo-gelling), the ability to create foams when aqueous solutions of the protein are whipped with air, or the ability to create emulsions when mixed with lipids in aqueous solutions of the protein. The term '*functional proteins'* as used herein is therefore considered similar to the terms *'soluble proteins',* '*(substantially) native proteins'* and '*(substantially) undenaturedproteins'* as used in the art.

The term '*insoluble fibers*', also denoted herein as *'FI',* means substances present in the potato fruit juice or in the derivatives thereof that can be separated from the liquid phase by centrifugation in a laboratory centrifuge at 4000 rpm for 30 minutes at room temperature. The precise chemical composition of the insoluble fibers may vary broadly, but may typically comprise insoluble polysaccharides, pectinates, starches and proteins and insoluble complexes of one or more of these substances.

The term '*absorbance at 600 nm'* as used herein relates to the amount of light of wavelength 600 nm passing through a liquid sample when measured in a spectrophotometer using 10 mm light path cuvettes. The absorbance of a liquid sample is often expressed as O.D. 600 nm. For the purpose of this disclosure, the absorbance is determined by spectrophotometry on samples diluted in 0.05 M potassium phosphate at pH 7.0, wherein, if need be, the pH is adjusted to 7.0 with sodium hydroxide or hydrochloric acid, to a read out in the range of 0.5 to 1.0, and this read out is multiplied with the dilution factor to determine the absorbance of the undiluted sample. If a sample has an absorbance of less than 1.0, no (substantial) dilution is performed, but the pH is nevertheless adjusted to 7.0 with sodium hydroxide or hydrochloric acid. In other words, the '*absorbance at 600 nm'* as used herein corresponds to the O.D.600 value of a sample as measured by spectrophotometry using 10 mm light path cuvettes on samples diluted in 0.05 M potassium phosphate at pH 7.0 to a read out in the range of 0.5 to 1.0 and the read out is multiplied with the dilution factor to determine the absorbance of the undiluted sample.

The term '*patatin*', also denoted herein as *'PA',* means storage glycoproteins found in potatoes *(Solanum tuberosum* L.). Patatin represents a group of immunologically identical glycoprotein isoforms with molecular masses in the range of 40-43 kDa. Patatin also has phospolipase activity capable of cleaving fatty acids from membrane lipids. For purposes of the invention, PA may be determined by different known assays, including SDS-PAGE combined with scanning densitometry (e.g. using a GS-900^{™} Calibrated Densitometer from BIO-RAD Laboratories, USA) including all protein bands in the molecular weight region between 35 kDa and 60 kDa in the PA category, ELISA testing using patatin specific antibodies, as well as enzymatic assays specific for the phospholipase activity (see e.g. M. Jiménez-Atiénzar et al., Determination of the phospholipase activity of patatin by a continuous spectrophotometric assay, Lipids, 2003, 38(6), pp 677-682).

The term '*protease inhibitor',* also denoted herein as '*PI*', means potato proteins, which possess molecular weights ranging from about 3 kDa to about 35 kDa, and which are capable of inhibiting the activity of *e.g.* serine proteases, cysteine proteases, aspartate proteases, and metalloproteases. For purposes of the invention PI may be determined by different known assays, including SDS-PAGE combined with scanning densitometry (e.g. using a GS-900^{™} Calibrated Densitometer from BIO-RAD Laboratories, USA) including all protein bands in the molecular weight region between 3 kDa and 35 kDa in the PI category, and more broadly by enzyme inhibition assays as generally described in the art (see e.g. R.E.J. Spelbrink et al., Quantitative Determination of Trypsin Inhibitory Activity in Complex Matrices, The Open Food Science Journal, 2011, 5, pp 42-46).

The term '*polyphenol oxidase',* also denoted herein as *'PPO',* means in the context of the present invention potato protein. Polyphenol oxidase (tyrosinase) (TY) is a bifunctional, copper-containing oxidase having both catecholase and cresolase activity. PPO causes the rapid polymerization of o-quinones to produce black, brown or red pigments (polyphenols) which cause fruit browning. The amino acid tyrosine contains a single phenolic ring that may be oxidised by the action of PPOs to form *o*-quinone. Hence, PPOs may also be referred to as tyrosinases. The catalytic action of PPO has a negative impact on the quality of several fruit and vegetable crops and results in alteration of color, flavor, texture, and nutritional value. It is a limiting factor in the handling and technological processing of crops as peeled, sliced, bruised or diseased tissues rapidly undergo browning. For purposes of the invention, PPO may be determined by different known assays as reviewed in: R. Yoruk et al., Physicochemical properties and function of plant polyphenol oxidase: A review, Journal of Food Biochemistry, 27(5), 2003, pp 361- 422.

The term '*lipoxygenase'*, also denoted herein as '*LipO*', means in the context of the present invention potato proteins capable of catalyzing the dioxygenation of polyunsaturated fatty acids. Lipoxygenases have food-related applications in bread making and aroma production but they also have negative implications for the color, off-flavour and antioxidant status of plant-based foods. In potatoes *(Solanum tuberosum* L.), lipoxygenase has a molecular weight of approximately 97 kD and can be detected by SDS-PAGE (see e.g. G. Bauw *et al.*, Patatins, Kunitz protease inhibitors and other major proteins in tuber of potato cv. Kuras, FEBS Journal, 2006, 273, pp 3569-3584). For purposes of the invention LipO may be determined by different known assays, including SDS-PAGE combined with scanning densitometry (e.g. using a GS-900^{™} Calibrated Densitometer from BIO-RAD Laboratories, USA) as well as enzyme activity assays as described in *e.g.* G.E. Anthon et al., Colorimetric method for the determination of lipoxygenase activity, J. Agric. Food Chem., 2001, 49, pp 32-37.

The term '*glycoalkaloids*' or '*alkaloid glucosides'* means a family of potentially toxic chemical compounds derived from alkaloids to which sugar groups are attached. Prototypical glycoalkaloids present in potatoes *(Solanum tuberosum* L.) are α-solanine and α-chaconine. In the context of the present disclosure, the level of '*total glycoalkaloids*' is expressed as the sum of α-solanine and α-chaconine. For purposes of the invention, glycoalkaloids may be determined by different known assays, including a standard HPLC assay as described in V. Alt et al., Optimization of glycoalkaloid analysis for use in industrial potato fruit juice downstreaming, Eng. Life Sci., 2005, 5, pp 562-567.

The term '*first salts'* means salts that are present in the liquid of the potato fruit juice of the derivative thereof at the outset of the separation process. Accordingly, the first salts include *'natural salts'* which are naturally present in potato fruit juice.

The term *'second salts'* concerns one or more salts added with water during the diafiltration step to increase the conductivity of the diafiltration liquid.

The term '*phenolic compounds*' means aromatic or heteroaromatic compounds comprising one or more ring systems and one or more phenolic hydroxyl groups. Phenolic compounds from potatoes include phenolics acids, flavonoids, tannins, stilbenes and lignans.

The term '*true protein concentration'* means the amount of protein per liter of a sample, calculated as the total weight or mass of amino acids per liter as determined by mesauring the total nitrogen in a sample by the method of Kjeldahl and using the conversion factor N x 6.25. All samples are dialyzed against demineralized water in dialysis tubing cellulose membrane (Sigma-Aldrich, USA, cat. No.: D9652) to remove any free amino acids and low molecular weight peptides prior to the nitrogen determination.

The term '*dry weight*' as used in the context of the present invention means the weight or mass of a substance remaining after removal of water by heating at 110 °C until the weight does not change anymore. The dry weight per ml sample is thus the remaining weight or mass of a sample of a substance having a volume of 1 ml before drying after removal of water by heating at 110 °C until the weight does not change anymore.

The term *'soluble'* as used in the context of the present invention refers to solubility of compounds in water at a concentration of at least 1 g/L at 25 °C.

The term '*diafiltration*' in the context of the present invention means a technique that uses membranes, typically ultrafiltration membranes, to completely remove, replace, or lower the concentration of salts or other low molecular weight substances from solutions containing proteins, peptides, nucleic acids, and other high molecular weight molecules. The process selectively utilizes permeable (porous) membrane filters to separate the components of solutions and suspensions based on their molecular size. An ultrafiltration membrane retains molecules that are larger than the pores of the membrane, while smaller molecules such as salts, polyphenols, solvents and water, may pass through the membrane. In a diafiltration process, water or a buffer composition *(i.e.* the diafiltration liquid) is continuously added to the retentate while the membrane filtration process continuously removes water, salts and low molecular weight compounds to the permeate side of the membrane.

The term *'nanofiltration'* means a membrane filtration-based method that uses nanometer sized through-pores across the membrane. Nanofiltration membranes typically have pore sizes from 1-10 nanometers, smaller than that used in microfiltration and ultrafiltration, but just larger than that used in reverse osmosis. Nanofiltration membranes are typically defined by the molecular weight cut-off (MWCO) of the membrane used. The nanofiltration membranes typically have a molecular weight cut-off of between 100 and 100 Da. Nanofiltration is applied in cross-flow mode under increased pressure.

The term *'ultrafiltration'* means a variety of membrane filtration processes in which forces like pressure or concentration gradients lead to a separation through a semipermeable membrane. Suspended solids and solutes of high molecular weight are retained in the so-called retentate, while water and low molecular weight solutes pass through the membrane in the permeate (filtrate). This separation process is typically used in industry and research for purifying and concentrating macromolecular solutions, especially protein solutions. Ultrafiltration membranes are defined by the pore size or by the molecular weight cut-off (MWCO) of the membrane used. Ultrafiltration membranes typically have a pore size of 0.01 - 0.1 µm or a molecular weight cut-off of between 1 and 500 kDa, such as between 5 and 50 kDa. Ultrafiltration is applied in cross-flow or dead-end mode. In a continuous process, ultrafiltration is applied in cross-flow mode.

The term '*microfiltration*' means a separation technique for removing micron-sized particles, like bacteria, suspended solids and colloid particles from liquid solutions. The process uses membrane filters with pores in the approximate size range of 0.1 to 10 µm, which are permeable to the fluid and dissolved substances, but retain certain particles, thus causing separation. Microfiltration membranes are defined by the nominal pore size of the membrane. Microfiltration is applied in cross-flow or dead-end mode. In a continuous process, microfiltration is applied in cross-flow mode.

The term *'turbidity'* means the measure of relative clarity of a liquid. It is an optical characteristic of water and is a measurement of the amount of light that is scattered by material in the water when light is shined through the water sample. The higher the intensity of scattered light, the higher the turbidity. Material that causes water to be turbid include clay, silt, very tiny particles of inorganic and organic matter and colloid fibers. Turbidity may be measured with a nephelometer. The units of turbidity from a calibrated nephelometer are called Nephelometric Turbidity Units (NTU).

The term '*mineral acids*' means inorganic acids, preferably the acids hydrochloric acid, nitric acid, phosphoric acid and sulfuric acid.

The term *'comprise'* and '*include*' as used throughout the specification and the accompanying items/claims as well as variations such as '*comprises*', *'comprising',* '*includes*' and '*including*' are to be interpreted inclusively. These words are intended to convey the possible inclusion of other elements or integers not specifically recited, where the context allows, unless specified otherwise.

The articles *'a*' and '*an*' are used herein to refer to one or to more than one *(i.e.* to one or at least one) of the grammatical object of the article. By way of example, '*an element'* may mean one element or more than one element, unless specified otherwise.

### BRIEF DESCRIPTION OF THE FIGURES

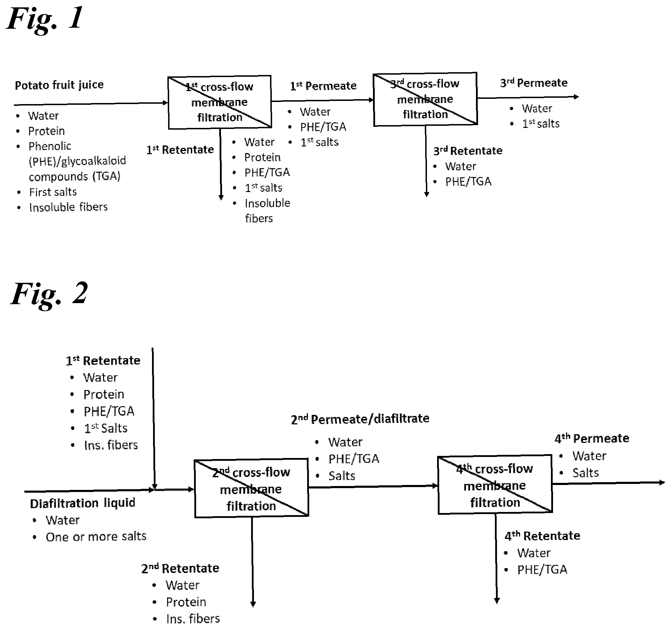

Figure 1 depicts a flow diagram of a preferred embodiment of the invention wherein potato fruit juice comprising insoluble fibers is subjected to a first cross-flow membrane filtration step, resulting in a first permeate and a first retentate comprising potato proteins and insoluble fibers (step (i) and (ii) of the process according to the invention), and wherein the first permeate is subjected to a third cross-flow membrane filtration step, resulting in a third permeate and a third retentate .
Figure 2 depicts a flow diagram of a preferred embodiment of the invention wherein the first retentate obtained in step (ii) is subjected in step (iii) to a second cross-flow membrane filtration step (diafiltration) using a diafiltration liquid and the resulting diafiltrate is subjected to a fourth cross-flow membrane filtration step, resulting in a fourth retentate enriched in phenolic and glycoalkaloid compounds and a fourth permeate comprising salts. The second retentate resulting from the a second cross-flow membrane filtration step (diafiltration) comprises potato proteins and insoluble fibers.

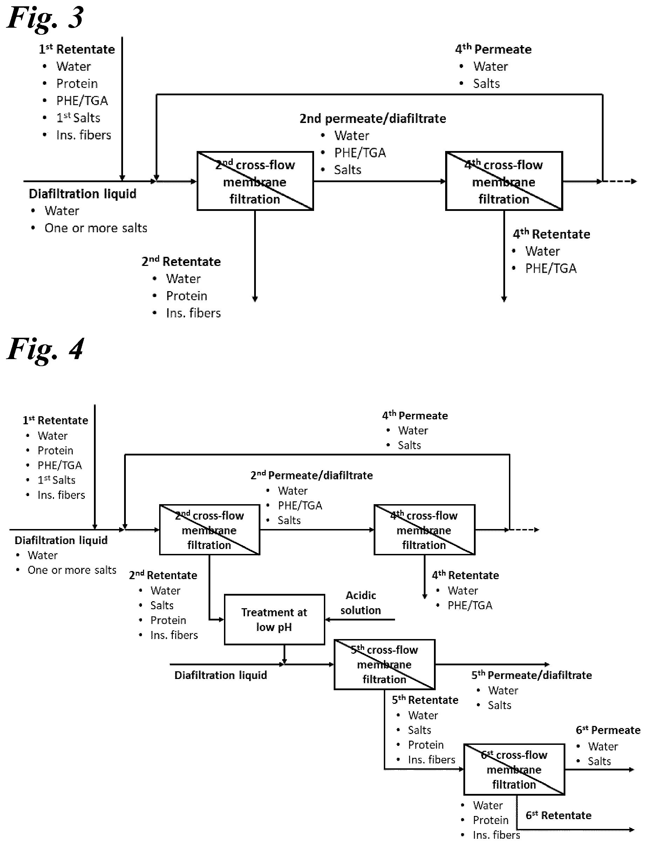

Figure 3 depicts a flow diagram of a preferred embodiment of the process depicted in Figure 2 wherein the fourth permeate comprising salts is recycled to the diafiltration liquid.
Figure 4 depicts a flow diagram of a preferred embodiment of the process depicted in Figure 3, wherein the second retentate comprising potato proteins and insoluble fibers is subjected to a step of inhibiting enzymatic activity using a temporary pH-drop, followed by diafiltration in a fifth cross-flow membrane filtration step resulting in a diafiltrate (fifth permeate) and a fifth retentate comprising potato proteins and insoluble fibers and by subjecting the fifth retentate to a sixth cross-flow membrane filtration step resulting in a sixth permeate mainly comprising water and a sixth retentate comprising potato proteins and insoluble fibers.

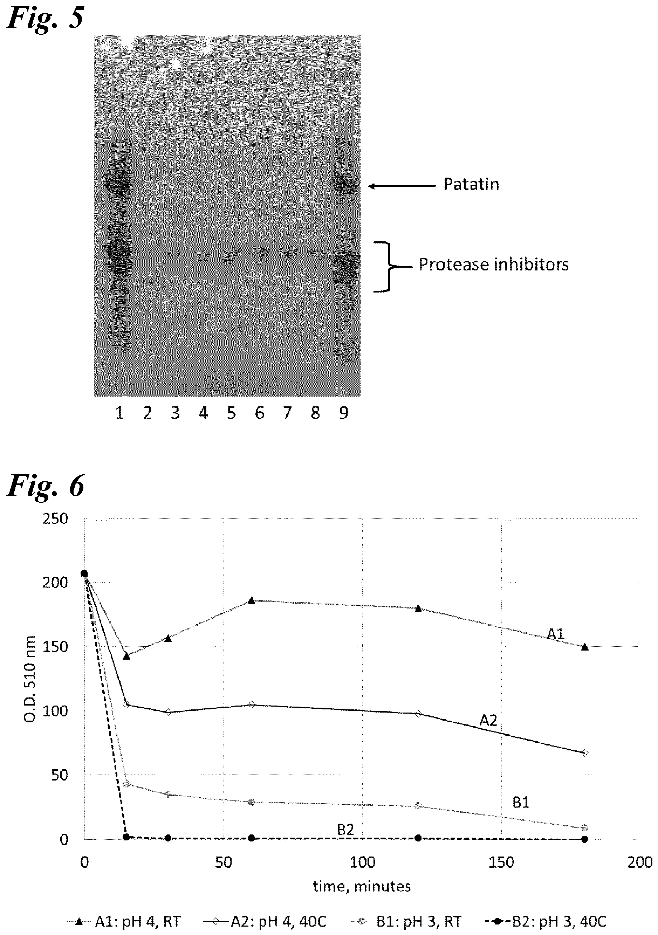

Figure 5 depicts SDS-PAGE analysis results of potato fruit juice, diafiltration permeates and the retentate after diafiltration obtained using a process according to the invention.
Figure 6 depicts esterase activity in potato protein retentate samples that were treated at different pH and temperature using a process according to the invention.

### DETAILED DESCRIPTION

### Method for separation of potato proteins and insoluble fibers from salts, phenolic compounds and/or glycoalkaloid compounds

In a first aspect, the present invention concerns a method for separation of (a) potato proteins and insoluble fibers from (b) first salts and phenolic and/or glycoalkaloid compounds in potato fruit juice or a derivative thereof, said method comprising the steps of:
(i) providing a potato fruit juice or a derivative thereof, comprising:
   - potato proteins; and
   - insoluble fibers; and
   - one or more first salts; and
   - phenolic and/or glycoalkaloid compounds,
   wherein the potato fruit juice or the derivative thereof has an absorbance at 600 nm in the range of 0.1 to 50, as determined using the method described (elsewhere) in the detailed description, and contains insoluble fibers at a concentration in the range of 0.1 to 30 mg/ml, based on the dry weight of the insoluble fiber;
(ii) subjecting the potato fruit juice or the derivative thereof provided in step (i) to a first cross-flow membrane filtration process wherein water and at least a portion of the first salts and at least a portion of the phenolic and/or glycoalkaloid compounds migrate across the membrane into a first permeate and wherein the potato proteins are retained in a first retentate, said first retentate having a true protein concentration in the range of 5 g/L to 200 g/L;
(iii) adding aqueous diafiltration liquid containing one or more salts to the first retentate obtained in step (ii) to form a diluted first retentate having a conductivity of between 0.1 mS/cm and 50 mS/cm and an absorbance at 600 nm of between 0.01 and 50 and subjecting said diluted first retentate to a second cross-flow membrane filtration as diafiltration, to create a second permeate being a diafiltrate containing at least a portion of said phenolic and/or glycoalkaloid compounds and salts and a second retentate comprising potato proteins, salts and insoluble fibers.

In a preferred embodiment, the process as defined herein is performed at a temperature in the range of 1 to 60 °C, such as 5 to 55 °C, 10 to 50 °C, 15 to 48 °C, 12 to 45 °C, 18 to 30 °C, or 22 to 28 °C.

In another preferred embodiment, the temperature of the first retentate and the second retentate is in the range of 1 to 60 °C, such as 5 to 55 °C, 10 to 50 °C, 15 to 48 °C, 12 to 45 °C, 18 to 30 °C, or 22 to 28 °C during step (ii) and step (iii).

In a very preferred embodiment, at least part of, more preferably all of the potato proteins remain in solution during the process steps as defined herein.

In a preferred embodiment, the first permeate from said first cross-flow membrane filtration process is subjected to a third cross-flow membrane filtration process, whereby at least a portion of the salts present in the first permeate migrate across the membrane into a third permeate and wherein the phenolic and/or glycoalkaloid compounds are retained in a third retentate

In this preferred embodiment, the phenolic and/or glycoalkaloid compounds are separated from the one or more first salts present in the first permeate such that the phenolic and/or glycoalkaloid compounds may be isolated in a purified state or a product enriched in the phenolic and glycoalkaloid compounds is obtained as a product of higher value.

In another preferred embodiment, the second permeate being a diafiltrate from said second cross-flow membrane filtration process is subjected to a fourth cross-flow membrane filtration process, whereby at least a portion of the salts present in said second permeate being a diafiltrate migrate across the membrane into a fourth permeate and wherein the phenolic and/or glycoalkaloid compounds are retained in a fourth retentate.

As a further highly important cost-saving step, the salts thus separated from the phenolic and/or glycoalkaloid compounds may then be recycled for use in the diafiltration step (iii). By doing so, there is not only saved a significant amount of water and salts for the process, but there will also be cost savings related to disposure of waste.

Accordingly, in a very preferred embodiment, the fourth permeate is used, at least in part, as the source of said aqueous diafiltration liquid containing one or more salts in claim 1, step (iii). In another preferred embodiment, the fourth permeate and the third permeate are used, at least in part, as the source of said aqueous diafiltration liquid containing one or more salts in step (iii).

In another embodiment, the fourth permeate is subjected to a reverse osmosis membrane filtration step or evaporation step and the resulting water is used, at least in part, as the source of the water in the aqueous diafiltration liquid in step (iii).

As will be appreciated by those skilled in the art, the second cross-flow membrane diafiltration process with this recycle stream can be performed in many ways. When starting the second cross-flow membrane diafiltration process, no fourth permeate is available yet. In a first embodiment, the diafiltration step (iii) starts with aqueous diafiltration liquid without added one or more salts and as soon as the fourth permeate is available from the fourth cross-flow membrane process step, this fourth permeate is recycled and used as aqueous diafiltration liquid in step (iii). In this first embodiment, the one or more salts in the aqueous diafiltration liquid thus solely originate from the one or more first salts. Alternatively, diafiltration step (iii) can be started by using the third permeate as aqueous diafiltration liquid and as soon as the fourth permeate is available from the fourth cross-flow membrane process step, this fourth permeate is recycled and used as aqueous diafiltration liquid. In a second embodiment, the diafiltration step (iii) starts with an aqueous diafiltration liquid containing one or more salts that are one or more second salts that are added to the aqueous diafiltration liquid and that are different from the one or more first salts.

Continuously adding fresh aqueous diafiltration liquid in step (iii) and recycling fourth and/or third permeate to the aqueous diafiltration liquid may require purging part of the fourth and/or third permeate to avoid buildup of the diafiltration flow. Continuously adding fresh aqueous diafiltration liquid in step (iii) and recycling fourth and/or third permeate to the diafiltration liquid combined with purging part of the fourth and/or third permeate may be advantageous to avoid buildup of too high concentrations of salts in the aqueous diafiltration liquid.

In a preferred embodiment, the method as defined herein is performed as a continuous process. As will be appreciated by the skilled person, if the method as defined herein is performed as a continuous process, the second cross-flow membrane device is positioned downstream of the first cross-flow membrane device, meaning that steps (ii) and (iii) are necessarily performed in different devices. Accordingly, in this embodiment, the first cross-flow membrane and the second cross-flow membrane can be different. In a preferred embodiment, the first and the second cross-flow membrane filtration processes are ultrafiltration processes, wherein the second cross-flow membrane filtration process is performed as diafiltration.

In another embodiment, the method as defined herein is performed as a (semi- )batch process wherein steps (ii) and (iii) are subsequently performed using the same cross-flow membrane. In this embodiment, the first and the second cross-flow membrane filtration processes are preferably ultrafiltration processes, wherein the second cross-flow membrane filtration process is performed as diafiltration.

In yet another embodiment, the method as defined herein is performed as a (semi- )batch process wherein steps (ii) and (iii) are subsequently performed using different cross-flow membranes, meaning that steps (ii) and (iii) are necessarily performed in different devices. Accordingly, in this embodiment, the first cross-flow membrane and the second cross-flow membrane can be different. In this embodiment, the first and the second cross-flow membrane filtration processes are preferably ultrafiltration processes, wherein the second cross-flow membrane filtration process is performed as diafiltration.

In an embodiment, the method as defined herein further comprises the step of subjecting the second retentate to a fifth membrane filtration process resulting in a fifth retentate, wherein at least a portion of the salts migrate across the membrane into a fifth permeate. This embodiment can also be performed as a diafiltration step. Accordingly, in an embodiment, aqueous diafiltration liquid is added to the second retentate to form a diluted second retentate before the fifth membrane filtration process, and the fifth membrane filtration process is performed on the diluted second retentate as diafiltration, resulting in a fifth permeate being a diafiltrate and the fifth retentate. In an embodiment, the membrane in the fifth membrane filtration process is an ultrafiltration membrane. In another embodiment, the membrane in the fifth membrane filtration process is a microfiltration membrane and at least a portion of the salts and the proteins soluble in the second retentate migrate across the microfiltration membrane.

In an embodiment, the fifth retentate is further treated with a sixth cross-flow membrane filtration process whereby the protein and insoluble fibers are concentrated in a sixth retentate and the salts migrate through the membrane to create a sixth permeate. The resulting sixth permeate comprising salts can be advantageously used as the aqueous diafiltration liquid in the fifth membrane filtration process, if performed as cross-flow diafiltration process. By doing so, there is not only saved a significant amount of water (and salts) for the process, but there will also be cost savings related to disposure of waste.

As will be appreciated by those skilled in the art, the fifth membrane filtration process and the sixth cross-flow membrane process do not separate potato proteins form insoluble fibers.

### Potato fruit juice or a derivative thereof

As explained hereinbefore, the term '*potato fruit juice or derivatives thereof*' means the liquid stream after the (industrial scale) separation of potato starch from potatoes *(solanum tuberosum* L.) and any aqueous streams derived from processing of such liquid stream by for example flocculation of contaminants and centrifugation, provided that the *'derivatives thereof*' still comprise:
- protein; and
- insoluble fibers; and
- salts; and
- phenolic and/or glycoalkaloid compounds.

The potato fruit juice or the derivative thereof that is used as a raw material in the process according to the present invention may be obtained by disintegration, e.g. by grinding, shredding and/or pressing, of the raw potatoes, whereby an aqueous solution comprising protein, salts, insoluble fibers, phenolic compounds and glycoalkaloid compounds is released as a juice, and/or the components may be extracted by addition of water or an aqueous extractant solution in combination with physical disruption of the plant tissue and/or cells. Antioxidants, such as sodium sulfite, sodium metabisulfite and ascorbic acid, may preferably be added prior to or during the procedures performed to produce the potato fruit juice or the derivative thereof. Likewise, it may be preferable to add salts to control conductivity and pH controlling substances such as acids or bases to adjust pH to a preferred value.

The potato fruit juice may be subjected to various pretreatment procedures creating a derivative of the potato fruit juice, such as deaeration, defoaming, solid-liquid separation (such as filtration, centrifugation, decantation, passing through hydroclyclones), heating or cooling.

The potato fruit juice or the derivative thereof so obtained may be pretreated in various ways prior to separation according to the invention. Preferably, the potato fruit juice or the derivative thereof is pretreated by centrifugation and/or filtration to remove insoluble material of a particle size larger than 10 micron prior to said first cross-flow membrane filtration process.

In a preferred embodiment the conductivity of the potato fruit juice or the derivative thereof provided in step (i) is in the range of 2-500 mS/cm, such as in the range of 1 to 50 mS/cm, 2 to 40 mS/cm, 3 to 30 mS/cm, 3 to 25 mS/cm, 3 to 20 mS/cm, or 5 to 17 mS/cm.

In another preferred embodiment, the method as defined herein further comprises the step of adjusting the conductivity of said potato fruit juice or the derivative thereof to a conductivity in the range of 2-500 mS/cm, such as in the range of 1 to 50 mS/cm, 2 to 40 mS/cm, 3 to 30 mS/cm, 3 to 25 mS/cm, 3 to 20 mS/cm, or 5 to 17 mS/cm prior to said first cross-flow membrane filtration process of step (ii).

In a preferred embodiment the pH of the potato fruit juice or the derivative thereof provided in step (i) is in the range of 4.5 to 8.5, such as in the range of 5.2 to 7.5. For certain applications, the pH of the potato fruit juice or the derivative thereof provided in step (i) is preferably in the range of 4.5 to 6.5, such as 4.8 to 6.5, or 5.0 to 6.5.

In another preferred embodiment, the method as defined herein further comprises the step of adjusting the pH of said potato fruit juice or the derivative thereof to a pH in the range of 4.5 to 8.5, such as in the range of 5.2 to 7.5 prior to said first cross-flow membrane filtration process of step (ii). For certain applications, the pH of the potato fruit juice or the derivative thereof is preferably adjusted to a pH in the range of 4.5 to 6.5, such as 4.8 to 6.5, or 5.0 to 6.5 prior to said first cross-flow membrane filtration process of step (ii).

### Potato proteins

In an embodiment, the potato proteins to be separated are selected from the group consisting of patatin, protease inhibitors, lipoxygenase, polyphenol oxidase, acid and alkaline phosphatase, or mixtures thereof, preferably patatin or protease inhibitors, or mixtures thereof.

In an embodiment, the potato protein to be separated is patatin. In another embodiment, the potato protein to be separated is protease inhibitors.

In a preferred embodiment, said potato proteins, said one or more first salts and said phenolic and/or glycoalkaloid compounds are in solution in said potato fruit juice provided in step (i).

### Phenolic and/or glycoalkaloid compounds

As explained hereinbefore, the potato fruit juice or the derivative thereof contains phenolic and/or glycoalkaloid compounds, such as (a) phenolic acids, flavonoids, tannins, stilbenes, lignans, gallic acid and/or catechin and (b) α-solanine and/or α-chaconine.

In an embodiment, the phenolic compounds to be separated are selected from the group consisting of phenolic acids and flavonoids including flavonols, flavanols, and anthocyanins. In another embodiment, the phenolic compounds to be separated are selected from the group consisting of phenolic acids and flavonoids including flavonols, flavanols, and anthocyanins, and combinations thereof.

In an embodiment, the phenolic compounds to be separated comprise chlorogenic acids. In another embodiment, the phenolic compounds to be separated comprise catechin.

In an embodiment, the glycoalkaloid compounds to be separated comprise α-solanine and/or α-chaconine.

In an embodiment, the phenolic and glycoalkaloid compounds to be separated are selected from the group consisting of (a) phenolic acids and flavonoids, including flavonols, flavanols, and anthocyanins, and (b) α-solanine and α-chaconine, and combinations thereof.

### Fibers

The potato fruit juice or the derivative thereof provided in step (i) contains insoluble fibers as defined hereinbefore.

The potato fruit juice or the derivative thereof provided in step (i) contains insoluble fibers at a concentration in the range of 0.1 to 30 mg/ml, based on the dry weight of the insoluble fiber, such as 0.2 to 20 mg/ml, 0.3 to 15 mg/ml, 0.5 to 10 mg/ml, or 1.0 to 5 mg/ml, based on the dry weight of the insoluble fiber.

The presence of insoluble fibers in the potato fruit juice can be related to its corresponding optical density or absorbance at a wavelength of 600 nm, which is a property that can be easily determined on a sample using spectroscopy. It is therefore specified, that the potato fruit juice or the derivative thereof has an absorbance at 600 nm in the range of 0.1 to 50.

In a preferred embodiment, the absorbance at 600 nm of the potato fruit juice or the derivative thereof is in the range of 0.2 to 45, such as in the range of 0.3 to 40, 0.4 to 35, 0.5 to 30, 0.7 to 25, 0.8 to 20, 0.9 to 15, 1.0 to 15, 1.5 to 15, or 2.0 to 15.

In an embodiment, more than 80 % of said insoluble fibers in the potato fruit juice or the derivative thereof are small enough to pass a 50 micron filter, such as a 40 micron filter, a 30 micro filter, a 20 micron filter, a 10 micron filter, a 5 micron filter, or a 2 micron filter.

In an embodiment, said insoluble fibers are present in the potato fruit juice in a concentration that results in a pellet when the fruit juice is centrifuged in a tabletop centrifuge at 4000 G for 30 min, wherein said pellet constitutes in the range of 0.1 to 10 vol/vol %, such as 0.1 to 5 vol/vol %, 0.1 to 3 vol/vol %, 0.1 to 2 vol/vol %, 0.1 to 1 vol/vol %, 0.2 to 5 vol/vol %, 0.2 to 3 vol/vol %, 0.3 to 5 vol/vol %, 0.3 to 3 vol/vol %, 0.4 to 5 vol/vol %, 0.4 to 4 vol/vol %, 0.5 to 5 vol/vol %, 0.5 to 4 vol/vol %, or 0.5 to 3 vol/vol %, when the potato fruit juice at a pH of 6 to 7 has been diluted (or concentrated) to a true protein concentration of 5 mg/ml.

### First cross-flow membrane process

In a preferred embodiment, the first cross-flow membrane filtration process to provide the first permeate and the first retentate according to step (ii) of the process as defined hereinbefore is an ultrafiltration process wherein high molecular weight compounds, such as proteins and insoluble fibers, are retained in the first retentate and low molecular weight substances, such as salts and phenolic and/or glycoalkaloid compounds migrate across the membrane as a first permeate.

Preferably the pore size of the ultrafiltration membrane is in the range of 5 to 500 kDa, such as 10 to 500 kDa, 10 to 200 kDa, 10 to 100 kDa, 10 to 50 kDa, 30 to 500 kDa, or 50 to 500 kDa.

More preferably, the pore size of the ultrafiltration membrane is in the range of 10 to 50 kDa, such as about 30 kDa.

The membrane material may be any kind of membrane suitable for cross-flow filtration, examples of which are polyamide, polysulfone, poyethersulfone, polyvinyl difluoride, polyacrylonitrile, cellulose acetate, permanently hydrophilized polysulfone, permanently hydrophilized polyethersulfone and ceramic membranes. Preferably, the membrane material is regenerated cellulose, polypropylene or polyethylene. Most preferably, the membrane material is selected from the group consisting of polysulfone, poyethersulfone, permanently hydrophilized polysulfone and permanently hydrophilized polyethersulfone.

In an embodiment, the membrane is a flat sheet or a spiral wound flat sheet membrane.

In an embodiment, the membrane is a hollow fiber or tubular membrane. In a preferred embodiment, the hollow fiber membrane has an internal diameter in the range of 0.5 to 3.0 mm, such as in the range of 0.6 to 2.5 mm, 0.7 to 2.2 mm, 0.8 to 2.0 mm, 0.9 to 1.8 mm, 1.0 to 1.7 mm, or 1.1 to 1.6 mm.

In a preferred embodiment, the hollow fiber membrane has a length in the range of 30 to 150 cm, such as in the range of 40 to 140 cm, 50 to 130 cm, 60 to 120 cm, 70 to 115 cm, 80 to 110 cm, or 90 to 110 cm.

In a preferred embodiment, the hollow fiber membrane material comprises polysulfone, cellulose acetate, ceramic material, polyamide, polyethersulfone or permanently hydrophilized polysulfone or polyethersulfone.

In a preferred embodiment, the hollow fiber membrane has a pore size in the range of 5 kDa to 500 kDa, such as in the range of 10 kDa to 200 kDa, in the range of 20 kDa to 100 kDa, or in the range of 30 kDa to 75 kDa.

In a preferred embodiment, the hollow fiber membrane is an inside-out membrane wherein the transmembrane transport of liquid is performed from the inner lumen of the membrane to the outside surface of the membrane.

Preferably, the conductivity of the first retentate remains within the range of 0.1 to 50 mS/cm, such as in the range of 1 to 40 mS/cm, 3 to 30 mS/cm, 3 to 25 mS/cm, 3 to 20 mS/cm, 4 to 20 mS/cm, 5 to 17 mS/cm, 6 to 25 mS/cm, 7 to 25 mS/cm, or 10 to 25 mS/cm during step (ii) as defined herein. More preferably, the electrical conductivity of the first retentate during the step (ii) remains within the range of 3 to 20 mS/cm.

Preferably, the pH of the first retentate remains within the range of 4.5 to 8.5, such as in the range of 5.2 to 7.5, preferably in the range of 4.5 to 6.5, 4.8 to 6.5, or 5.0 to 6.5 during step (ii) as defined herein.

Preferably, the temperature of the first retentate is in the range of 1 to 60 °C, such as 5 to 55 °C, 10 to 50 °C, 15 to 48 °C, 12 to 45°C, 18 to 30 °C, or 22 to 28 °C during step (ii) as defined herein.

In a preferred embodiment, the absorbance at 600 nm of the first retentate remains within the range of 0.01 to 100, such as in the range of 0.1 to 100, 0.5 to 100, 1.0 to 100, 2.0 to 80, 3.0 to 70, 4.0 to 60, or 5.0 to 50 during step (ii) as defined herein. Since the first retentate is concentrated as compared to the potato fruit juice or the derivative thereof provided in step (i), the absorbance at 600 nm of the first retentate is typically higher than that of the potato fruit juice or the derivative thereof.

In a preferred embodiment, the true protein concentration of the first retentate is in the range of 8 g/L to 180 g/L, such as in the range of 10 g/L to 160 g/L, 12 g/L to 150 g/L, 15 g/L to 150 g/L, 20 g/L to 150 g/L, 25 g/L to 150 g/L, 30 g/L to 150 g/L, 20 g/L to 140 g/L, 20 g/L to 130 g/L, 20 g/L to 120 g/L, 20 g/L to 100 g/L, 20 g/L to 80 g/L, 20 g/L to 60 g/L, or 30 g/L to 60 g/L.

In another preferred embodiment, the solubility of the potato protein in the first retentate obtained in step (ii) remains within the range of 50 % to 99 %, such as in the range of 55 % to 98 %, 60 % to 98 %, 65 % to 98 %, 70 % to 98 %, 75 % to 98 %, 80 % to 98 %, or 85 % to 98 %, relative to the protein soluble in the potato fruit juice or the derivative thereof.

### Third and fourth cross-flow membrane filtration processes

In a preferred embodiment, the third and/or fourth cross-flow membrane filtration processes as defined hereinbefore are nanofiltration processes wherein phenolic and/or glycoalkaloid compounds are retained in the retentate and (first) salts migrate across the membrane as a permeate.

Preferred materials for the nanofiltration membrane include organic polymers, such as cellulose acetate, polysulfone, poly(ether)sulfone, sulfonated polysulfone, aromatic polyamides, polyimide, poly(piperazine amide) and blends and composites thereof.

The pore size of the nanofiltration membrane is preferably in the range of 100-1000 Da, such as 200-800 Da, or 300-700 Da. For some applications, the pore size is preferably in the range of 500-800 Da, such as 300-500 D, or 150-300 Da.

Especially interesting nanofiltration devices for the separation of polyphenols and/or glycoalkaloids are Filmtec NF elements from Dupont, USA, Duracon and Dairy Thin film NF elements from SUEZ Water Technologies & Solutions, USA, NFX, NFW and NFG elements from Synder Filtration, USA, vNF Spiral thin film composite membranes from Alfa Laval Corp, Sweden, and X-Flow hollow fiber nanofiltration elements from Pentair, The Netherlands.

### Diafiltration step

In order to optimize the processing logistics, and for some applications the processability of said first retentate, the first retentate obtained in step (ii) may advantageously be stored for several hours before the diafiltration of step (iii) is carried out. Thus, in an embodiment, said first retentate is stored for at least 3 hours, such as at least 6 hours, at least 12 hours, at least 18 hours, at least 24 hours, at least 36 hours, or at least 48 hours before the diafiltration in the second cross-flow membrane filtration step of (iii) is performed. In a further embodiment, the diafiltration in the second cross-flow membrane filtration step (iii) may be partly carried out before the storage of said first retentate.

In yet a further embodiment, said first retentate obtained in step (ii) may be treated with a preliminary diafiltration step using e.g. water or a low conductivity aqueous solution prior to storage and subsequent diafiltration according to step (iii).

In an embodiment, the storage temperature of said first retentate is within the range of 1 to 25 °C, such as 2 to 20 °C, 3 to 15 °C, 2 to 12 °C, 2 to 10 °C, or 2 to 8 °C.

In a further embodiment, the pH of said first retentate during storage is, or is adjusted to, a pH in the range of 4.5 to 5.6, such as 4.5 to 5.4, 4.5 to 5.2, or 4.7 to 5.3.

The one or more second salts that may be added to the aqueous diafiltration liquid in step (iii) of the method as defined herein may be any water soluble salt of on the one hand one or more of sodium, potassium, ammonium, calcium and magnesium and on the other hand one or more of mineral acids and organic acids.

In a preferred embodiment, the one or more second salts are selected from sodium, potassium, ammonium, calcium or magnesium salts of a mineral acid or an organic acid.

Examples of suitable organic acids are formic acid, acetic acid, propanoic acid, citric acid, caprylic acid, lactic acid, gluconic acid, tartaric acid, fumaric acid, butanedioic acid and benzoic acid.

As explained hereinbefore, the membrane applied in the fourth cross-flow membrane filtration step preferably is a nanofiltration membrane. In order to achieve the highest permeability of the salt through a nanofiltration membrane retaining the phenolic compounds in the retentate, the one or more second salts are preferably chosen from the group of salts constituted by only monovalent inorganic anions and cations, such as sodium chloride, potassium chloride and ammonium chloride.

However, in certain applications there will be a need to limit process costs and the cost of environmentally friendly waste handling. Likewise, certain factory designs cannot withstand the corrosive nature of *e.g.* chlorides. For such applications, the one or more salts are preferably chosen from the group of sodium sulfate and potassium sulfate.

The amount of the one or more (second) salts in (or added to) the aqueous diafiltration liquid determines the conductivity of the diluted first retentate during diafiltration step (iii).

As explained hereinbefore, the diluted first retentate has a conductivity of between 0.1 mS/cm and 50 mS/cm.

In a preferred embodiment, the electrical conductivity of the second retentate (or the diluted first retentate) remains within the range of 0.1 to 50 mS/cm, such as in the range of 1 to 40 mS/cm, 3 to 30 mS/cm, 3 to 25 mS/cm, 3 to 20 mS/cm, 4 to 20 mS/cm, 5 to 17 mS/cm, 6 to 25 mS/cm, 7 to 25 mS/cm, or 10 to 25 mS/cm during step (iii).

More preferably, the electrical conductivity of the second retentate (or the diluted first retentate) during the diafiltration step (iii) remains within the range of 3 to 20 mS/cm.

The nature of the one or more second salts that may be added to the aqueous diafiltration liquid in step (iii) may also influence the pH of the diluted first retentate and the second retentate. In a preferred embodiment the pH of the second retentate (or the diluted first retentate) remains within the range of 4.5 to 8.5, such as in the range of 5.2 to 7.5, preferably in the range of 4.5 to 6.5, 4.8 to 6.5, or 5.0 to 6.5 during step (iii).

Preferably, the temperature of the second retentate (or the diluted first retentate) is in the range of 1 to 60 °C, such as 5 to 55 °C, 10 to 50 °C, 15 to 48 °C, 12 to 45°C, 18 to 30 °C, or 22 to 28 °C during step (iii) as defined herein.

In a particular embodiment, the diafiltration in step (iii) is performed first at a relatively low temperature to remove the majority of the phenolic and/or glycoalkaloid compounds followed by diafiltration at a relatively higher temperature to remove the remaining phenolic and/or glycoalkaloid compounds. Thus, in a preferred embodiment the retentate in a first phase of diafiltration remains at a temperature in the range of 1 to 30 °C, such as 5 to 28 °C, 10 to 25 °C, or 15 to 22 °C, followed by a second phase of diafiltration wherein the retentate remains at a temperature in the range of 30 to 60 °C, such as 30 to 55 °C, 35 to 52 °C, 38 to 50 °C, or 40 to 48 °C.

The addition of the aqueous diafiltration liquid step (iii) is to be performed until enough volume has been added, and subsequently removed via the second permeate, to reach a satisfactory (low) level of phenolic and/or glycoalkaloid compounds in the second retentate.

In an embodiment, the addition of aqueous diafiltration liquid containing one or more salts to the first retentate is continued while continuing the second membrane filtration process in step (iii) until the second retentate contains less than a target amount of the phenolic and/or total glycoalkaloid compounds, whereby the separation of the potato proteins from phenolic and/or total glycoalkaloid compounds has been achieved, wherein the target amount of remaining phenolic and/or total glycoalkaloid compounds in the second retentate corresponds to less than 5000 mg phenolic and/or total glycoalkaloid compounds per kg potato protein on the basis of dry weight, such as less than 4000 mg/kg, less than 3000 mg/kg, less than 2000 mg/kg, less than 1500 mg/kg, less than 1250 mg/kg, less than 1000 mg/kg, less than 750 mg/kg, less than 500 mg/kg, or less than 200 mg phenolic and/or total glycoalkaloid compounds/kg potato protein on the basis of dry weight.

In some applications it is further important to reach a lower level of phenolic and/or total glycoalkaloid compounds having a molecular weight below 10 kDa. Thus, in a further embodiment, the target value for phenolic and/or total glycoalkaloid compounds having a molecular weight below 10 kDa remaining in the second retentate of diafiltration step (iii) corresponds to less than 1500 mg/kg, such as less than 1000 mg/kg, less than 750 mg/kg, less than 500 mg/kg, less than 300 mg/kg, less than 200 mg/kg, less than 100 mg/kg, or less than 50 mg phenolic and/or total glycoalkaloid compounds/kg potato protein on the basis of dry weight.

In an embodiment, glycoalkaloids are efficiently separated from the potato proteins and insoluble fibers present in the potato fruit juice or the derivative thereof. Thus, in an embodiment, diafiltration step (iii) brings the total glycoalkaloids remaining in the second retentate below a target amount corresponding to less than 500 mg/kg, such as less than 300 mg/kg, less than 200 mg/kg, less than 100 mg/kg, or less than 50 mg total glycoalkaloids/kg potato protein on the basis of dry weight.

In an embodiment, the addition of aqueous diafiltration liquid in step (iii) is performed continuously and with practically the same flow rate as the diafiltrate is migrating through the second cross-flow membrane. In another embodiment, the addition of aqueous diafiltration liquid in step (iii) is done in small portions, such as portions approximately equal to the retentate volume.

In an embodiment, the total volume of water added during diafiltration step (iii) is in the range of 4 to 20 times the volume of the first retentate, such as 4 to 15 times, 5 to 12 times, 5 to 10 times, 6 to 10 times, 7 to 9 times, or 5 to 9 times the volume of the first retentate. More preferably, the total volume of water added during diafiltration step (iii) is in the range of 6 to 10 times the volume of the first retentate.

In a preferred embodiment, diafiltration step (iii) comprises a second phase of diafiltration following the addition of aqueous diafiltration liquid containing one or more salts, wherein the diafiltration is continued with the addition of water without salts. In this way the salt concentration and conductivity of the second retentate (or the diluted first retentate) will gradually decrease until a target value for the conductivity has been reached.

In an embodiment, the second phase of diafiltration in step (iii) is continued with water and without the addition of salts until the conductivity of the second retentate is less than 10 mS/cm, such as less than 8 mS/cm, less than 5 mS/cm, less than 4 mS/cm, less than 3 mS/cm, less than 2 mS/cm, or less than 1 mS/cm.

In a preferred embodiment, the absorbance at 600 nm of the second retentate (or the diluted first retentate) remains within the range of 0.01 to 100, such as in the range of 0.1 to 100, 0.5 to 100, 1.0 to 100, 2.0 to 80, 3.0 to 70, 4.0 to 60, or 5.0 to 50 during step (iii) as defined herein.

In another preferred embodiment, the solubility of the potato protein in the second retentate obtained in step (iii) remains within the range of 50 % to 99 %, such as in the range of 55 % to 98 %, 60 % to 98 %, 65 % to 98 %, 70 % to 98 %, 75 % to 98 %, 80 % to 98 %, or 85 % to 98 % relative to the protein soluble in the potato fruit juice or the derivative thereof.

In order to optimize the processing logistics, and for some applications the processability of the retentate resulting after diafiltration step (iii), the second retentate may advantageously be stored for several hours before further processing.

Thus, in an embodiment, the second retentate resulting after the diafiltration step (iii) is stored for at least 3 hours, such as at least 6 hours, at least 12 hours, at least 18 hours, at least 24 hours, at least 36 hours, or at least 48 hours before further processing is performed.

In an embodiment, the storage temperature of said second retentate resulting after diafiltration step (iii) is within the range of 1 to 25 °C, such as 2 to 20 °C, 3 to 15 °C, 2 to 12 °C, 2 to 10 °C, or 2 to 8 °C.

In a further embodiment, the pH of said second retentate resulting after diafiltration step (iii) during storage is, or is adjusted to, a pH in the range of 4.5 to 5.6, such as 4.5 to 5.4, 4.5 to 5.2, or 4.7 to 5.3.

### Inactivation of microbial growth during processing

Potato fruit juice and other extracts of potato proteins may contain a high level of microbes naturally originating from the growth and storage of the potatoes in the soil. It is therefore preferred to add a germicidal step during the process according to the invention. This step may be applied directly to the crude potato fruit juice, or it may preferably be applied during and/or in between the membrane filtration processing steps according to the invention.

Thus, in an embodiment, the germicidal step is performed on the potato fruit juice or the derivative thereof provided in step (i) prior to step (ii). Before diafiltration in step (iii) and after the concentration in step (ii), the volumetric flow rate is reduced. Accordingly, it may be advantageous to perform the germicidal step just before step (iii). Thus, in a preferred embodiment, the germicidal step is performed on the first retentate provided in step (ii) prior to diluting with the aqueous diafiltration liquid in step (iii).

Since many proteins are highly sensitive to classical pasteurization by heat treatment, leading to loss of functional properties of the proteins, the germicidal step is performed at a temperature in the range of 2-60 °C, such as in the range of 5-55 °C, in the range of 8-52 °C, in the range of 10-50 °C, in the range of 12-45 °C, in the range of 12-25 °C, or in the range of 12-18 °C.

The germicidal step is applied using high intensity UV light, X-rays, pulsed electric field treatment or high-pressure pasteurization.

In an embodiment, the germicidal step comprises passing of the potato fruit juice or the derivative thereof through a photo bioreactor illuminating high intensity UV light to the fruit juice. In an embodiment, the UV light has a wavelength primarily in the range of 180-300 nm. In an embodiment, the potato fruit juice or the derivative thereof is illuminated with UV light when passing one or more a spiral-shaped tubes, allowing the passage of the UV light. In an embodiment, the inner diameter of said spiral tubes is in the range of 1-15 mm, such as in the range of 2-12 mm, such as in the range of 3-10 mm, such as in the range of 4-9 mm. In an embodiment, the potato fruit juice or the derivative thereof is passed more than one time through the photo bioreactor.

In an embodiment, the potato fruit juice or the derivative thereof is passing the photo bioreactor simultaneously with one or more of the membrane filtration steps according to the invention. In an embodiment, the photo bioreactor is inserted into the recirculation loop of one or more of the membrane filtration units applied according to the invention such that the potato fruit juice or the derivative thereof is continuously passing the photo bioreactor.

In an embodiment, the photo bioreactor is inserted as a shunt to the recirculation loop of one or more of the membrane filtration units applied according to the invention. In an embodiment, the photo bioreactor is inserted as a recirculation unit to the holding tank of one or more of the membrane filtration units applied according to the invention. In an embodiment, the germicidal step using a photo bioreactor is applied to the protein solution after the membrane filtration steps according to the invention. In an embodiment, the O.D.600 nm absorption of the resulting protein solution is in the range of 0.02 to 15.0, such as in the range of 0.05 to 8.0, in the range of 0.1 to 7.0, in the range of 0.5 to 6.0, in the range of 0.8 to 5.0, in the range of 0.9 to 4.0, or in the range of 1.0 to 3.0.

In an embodiment of the invention the photo bioreactor is substantially equal to the photo bioreactor disclosed in WO2019/057257A1.

### Inactivation of unwanted enzymatic activities

In a preferred embodiment, the first retentate obtained in step (ii) prior to step (iii) or the potato proteins with insoluble fibers isolated according to the invention are treated to provide inactivation of enzymatic activities that may be unwanted when the proteins are to be used as food ingredients in certain applications. In particular, inactivation of the esterase and lipolytic activity of patatins and the inactivation of polyphenol oxidase activity is preferred. It is well known that such inactivation may be performed by extensive heating of the proteins. However, such treatment may also lead to uncontrolled polymerization and gelling/denaturation of the proteins, such that the functionality for use in foods is lost or at least significantly decreased.

The inventors have surprisingly found that treatment of the first retentate obtained in step (ii) prior to step (iii) or the mixtures of potato proteins and fibers isolated according to the invention with acidic pH values for a limited period of time may lead to the preferred inactivation, particularly inactivation of the esterase and lipolytic activity of patatins, in a controlled manner without losing the functionality of the proteins (after raising the pH again). Moreover, the inventors have surprisingly found that this inactivation, particularly inactivation of the esterase and lipolytic activity of patatins, improves the flavor of the mixtures of potato proteins and fibers isolated according to the invention.

The wording *'or the potato proteins with insoluble fibers isolated according to the invention'* refers to any one of the following:
- the second retentate resulting from diafiltration step (iii);
- the fifth retentate resulting from the fifth cross-flow membrane filtration step;
- the sixth retentate resulting from the sixth cross-flow membrane filtration step; or
- any other aqueous stream comprising potato proteins and insoluble fibers obtained downstream of diafiltration step (iii).

Thus, in a preferred embodiment, the first retentate obtained in step (ii) prior to step (iii) or a solution of the potato proteins with insoluble fibers isolated according to the invention, preferably the second retentate resulting from diafiltration step (iii), is exposed to pH values between 2.0 and 4.5, such as between 2.0 and 4.0, between 2.0 and 3.75, between 2.0 and 3.5, between 2.0 and 3.2, or between 2.0 and 3.0 in a time and temperature interval sufficient to eliminate at least part of the unwanted enzymatic activity of the potato proteins without adversely affecting functionality of the potato proteins.

As will be appreciated by those skilled in the art, the wording '*without adversely affecting functionality of the potato proteins'* is to be understood as *'without substantially adversely affecting functionality of the potato proteins'* because subjecting the proteins to the acidic pH values for a limited period of time may negatively affect at least part of the functionality of the proteins.

In an embodiment, the first retentate obtained in step (ii) prior to step (iii) or the potato protein solution with insoluble fibers isolated according to the invention, preferably the second retentate resulting from diafiltration step (iii), exposed to the low pH comprises patatin and the enzymatic activity to be eliminated is the esterase activity of said patatin.

In an embodiment, the first retentate obtained in step (ii) prior to step (iii) or the potato protein solution with insoluble fibers isolated according to the invention, preferably the second retentate resulting from diafiltration step (iii), exposed to the low pH comprises one or more polyphenol oxidases and the enzymatic activity to be eliminated is the oxidative activity of said polyphenol oxidase.

In an embodiment, the first retentate obtained in step (ii) prior to step (iii) or the potato protein solution with insoluble fibers isolated according to the invention, preferably the second retentate resulting from diafiltration step (iii), exposed to the low pH comprise phospholipase and the enzymatic activity to be eliminated is the phospholipase activity of said phospholipase.

In an embodiment, the inactivation of enzymatic activity is performed at a temperature in the range of 1-70 °C, such as in the range of 1-65 °C, 5-60 °C, 5-15 °C, 10-55 °C, 10-15 °C, 15-55 °C, 18-50 °C, 18-48 °C, or 20-45 °C.

In an embodiment, the inactivation of enzymatic activity is performed in a time span of at least 15 seconds, such as at least 5 minutes, such as at least 10 minutes, such as at least 30 minutes, such as at least 60 minutes, such as at least 120 minutes, such as at least 240 minutes.

In a very preferred embodiment, the inactivation of enzymatic activity is performed by exposing the first retentate obtained in step (ii) prior to step (iii) or a solution of the potato proteins with insoluble fibers isolated according to the invention, preferably the second retentate resulting from diafiltration step (iii), to:
- a pH value between 2.0 and 4.5, preferably between 2.0 and 4.0, more preferably between 2.0 and 3.75, even more preferably between 2.0 and 3.5, such as between 2.0 and 3.2, or between 2.0 and 3.0;
- a temperature in the range of 1-70 °C, preferably in the range of 5-60 °C, more preferably in the range of 10-55 °C, even more preferably in the range of 20-45 °C; and
- in a time span of up to 240 minutes, preferably between 15 seconds and 120 minutes, more preferably between 5 and 60 minutes, such as about 15, 20, 30 or 45 minutes.

In a preferred embodiment, the pH of the thus treated stream is brought back to a value between 4.5 and 8.5, such as between 6 and 8, or between 6.5 and 7.5. As will be appreciated by those skilled in the art, such an additional step is not needed if the step of inactivation of enzymatic activity is performed just before a diafiltration step wherein aqueous diafiltration liquid is added, automatically resulting in an increase of the pH.

The inactivation of enzymatic activity is preferably performed by exposing the first retentate obtained in step (ii) before diafiltration step (iii) to the low pH. Before diafiltration in step (iii) and after the concentration in step (ii), the volumetric flow rate is reduced, such that the amount of acid needed to reduce the pH is limited. Moreover, by subjecting thus treated retentate to diafiltration in step (iii), the pH can be quickly brought back to a value of 4.5 or higher to avoid overexposure of the proteins.

More preferably, the inactivation of enzymatic activity is performed by exposing the second retentate resulting from diafiltration step (iii) to the low pH. Since the polyphenols have been sufficiently removed from this stream, the risk of unwanted chemical reactions at acidic pH is eliminated.

It was further surprisingly found that a controlled and limited proteolysis of the potato proteins isolated according to the invention may lead to inactivation of unwanted enzymatic activity without destroying important functional properties, such as the ability to form food gels.

Thus, in an embodiment, the inactivation of unwanted enzymatic activity of the potato protein isolated according to the invention is achieved by treatment of a solution of the potato protein with insoluble fibers with one or more proteolytic enzymes. In an embodiment, the enzymatic activity of patatin is in part or fully inactivated by the treatment with a protease. In an embodiment, the enzymatic activity of phospholipase is in part or fully inactivated by the treatment with a protease. In an embodiment, the enzymatic activity of polyphenol oxidase is in part or fully inactivated by the treatment with a protease.

In an embodiment, the one or more proteases are endopeptidase. In an embodiment, the one or more proteases are serine proteases. In an embodiment, the one or more proteases are chosen from the group of trypsin-like, chymotrypsin-like, thrombin-like, elastase-like and subtilisin-like proteases. In an embodiment, the one or more proteases are subtilisin.

### Potato fruit juice product

In a second aspect (not claimed), the invention concerns a potato fruit juice product, comprising protein and insoluble fibers obtainable or obtained by via the process as defined herein.

As will be appreciated by those skilled in the art, the wording '*potatofruit juice product, comprising protein and insoluble fibers obtainable or obtained by via the process as defined herein'* refers to any one of the following:
- the second retentate resulting from diafiltration step (iii);
- the fifth retentate resulting from the fifth cross-flow membrane filtration step;
- the sixth retentate resulting from the sixth cross-flow membrane filtration step; or
- any other aqueous stream comprising potato proteins and insoluble fibers obtained downstream of diafiltration step (iii).

This potato fruit juice product can be distinguished from potato fruit juice products disclosed in the prior art in that it comprises a purified mixture of functional potato protein with an increased amount of insoluble fibers and a very low amount of salts, phenolic compounds and glycoalkaloid compounds.

**In** a preferred embodiment, the potato fruit juice product has a true protein content of at least 60%, such as at least 65 %, at least 70 %, at least 75 %, at least 80 %, at least 85 %, or at least 90 %.

**In** another preferred embodiment, the potato fruit juice product has a content of insoluble fibers in the range of 2 to 39 %, such as in the range of 5 to 30 %, 7 to 25 %, 5 to 12 % 5 to 10 %, 5 to 8 %, 2-5%, or 3-8 % on a dry weight basis.

**In** yet another preferred embodiment, more than 80 % of said insoluble fibers in the potato fruit juice product are small enough to pass a 50 micron filter, such as a 40 micron filter, a 30 micron filter, a 20 micron filter, a 10 micro filter, a 5 micron filter, or a 2 micron filter.

**In** yet another preferred embodiment, the amount of phenolic compounds in the potato fruit juice product corresponds to less than 5000 mg phenolic compounds per kg potato protein on the basis of dry weight, such as less than 4000 mg/kg, less than 3000 mg/kg, less than 2000 mg/kg, less than 1500 mg/kg, less than 1250 mg/kg, less than 1000 mg/kg, less than 750 mg/kg, less than 500 mg/kg, or less than 200 mg phenolic compounds/kg potato protein on the basis of dry weight.

**In** yet another preferred embodiment, the amount of total glycoalkaloid compounds in the potato fruit juice product is less than 500 mg/kg, such as less than 300 mg/kg, such as less than 200 mg/kg, such as less than 100 mg/kg, such as less than 50 mg/kg, measured as mg total glycoalkaloid compounds/kg potato protein on the basis of dry weight.

**In** yet another preferred embodiment, the water content in the potato fruit juice product is less than 10%, such as less than 7 %, less than 6 %, less than 5 %, or less than 4 %.

**In** yet another preferred embodiment, the potato fruit juice product is an aqueous suspension or a paste of said potato protein and insoluble fibers having a dry matter content of 0.1 % to 80 %, such as 0.5 % to 75 %, 1 % to 73 %, 2 % to 65 %, 5 % to 60 %, 10 % to 50 %, 10 % to 40 %, 10 % to 30 %, or 15 % to 28 %.

**In** a very preferred embodiment, the potato fruit juice product as defined herein has one or more, preferably all, of the following microbial requirements:
- total aerobic count: ≤ 10⁴ CFU/(g of the potato fruit juice product);
- yeasts: ≤ 100 CFU/(g of the potato fruit juice product);
- moulds: ≤ 100 CFU/(g of the potato fruit juice product);
- *B. cereus:* ≤ 100 CFU/(g of the potato fruit juice product).

More preferably, the potato fruit juice product as defined herein has one or more, preferably all, of the following microbial requirements:
- total aerobic count: ≤ 10³ CFU/(g of the potato fruit juice product);
- yeasts: ≤ 50 CFU/(g of the potato fruit juice product);
- moulds: ≤ 50 CFU/(g of the potato fruit juice product);
- *B. cereus:* ≤ 50 CFU/(g of the potato fruit juice product).

Even more preferably, the potato fruit juice product as defined herein has one or more, preferably all, of the following microbial requirements:
- total aerobic count: ≤ 10³ CFU/(g of the potato fruit juice product);
- yeasts: ≤ 10 CFU/(g of the potato fruit juice product);
- moulds: ≤ 10 CFU/(g of the potato fruit juice product);
- *B. cereus:* ≤ 10 CFU/(g of the potato fruit juice product).

**In** a third aspect (not claimed), the invention concerns a potato fruit juice product, comprising protein and insoluble fibers, having:
- a true potato protein content of at least 60%, such as at least 65 %, at least 70 %, at least 75 %, at least 80 %, at least 85 %, or at least 90 %;
- a content of insoluble fibers in the range of 2 to 39 %, such as in the range of 5 to 30 %, 7 to 25 %, 5 to 12 % 5 to 10 %, 5 to 8 %, 2-5%, or 3-8 % on a dry weight basis;
- an amount of phenolic compounds of less than 5000 mg phenolic compounds per kg potato protein on the basis of dry weight, such as less than 4000 mg/kg, less than 3000 mg/kg, less than 2000 mg/kg, less than 1500 mg/kg, less than 1250 mg/kg, less than 1000 mg/kg, less than 750 mg/kg, less than 500 mg/kg, or less than 200 mg phenolic compounds/kg potato protein on the basis of dry weight; and
- an amount of total glycoalkaloid compounds of less than 500 mg/kg, such as less than 300 mg/kg, such as less than 200 mg/kg, such as less than 100 mg/kg, such as less than 50 mg/kg, measured as mg total glycoalkaloid compounds/kg potato protein on the basis of dry weight.

Further preferred embodiment described in the context of the second aspect also apply to the potato fruit juice product according to the third aspect.

### EXAMPLES

### Materials

Chemicals used in the examples herein *e.g.* for preparing buffers and solutions are commercial products of at least reagent grade. Water used for conducting the experiments is all de-ionized water.

### Potato fruit juice and derivatives thereof

Potatoes of the variety Folva were obtained from a local supermarket. The potatoes were washed and their surfaces were dried off before the potatoes were shredded including the peel, while the liberated liquid (juice) concomitantly was separated from the main mass of insolubles using a commercial juicer (Nutrijuicer PRO) without diluting with water. A volume of 10 ml sodium sulphite (10 wt%) per 1000 ml juice was added immediately to the juice and the juice was centrifuged for 5 min at 1430 G to remove any remaining large insoluble particles such as insoluble fibers and starch.

An amount of 10 kg of potatoes yielded about 4.65 L of centrifuged juice with a pH of 6.2 and a conductivity of 10.7 mS/cm, as measured with a Seven2Go S3 conductivity meter from Mettler Toledo, Switzerland. The so obtained potato fruit juice did not contain visible particles larger than about 10 µm, but nevertheless remained unclear with suspended colloid particles.

### Buffer solutions

(I) A 10 wt% sodium sulphite buffer solution was prepared by dissolving 10 g of sodium sulphite from Sigma Aldrich USA (cat. No.: 13471) in water to a total volume of 100 ml;
(II) A 0.1 M di-potassium hydrogen phosphate pH 7.0 buffer solution was prepared by dissolving 17.42 g dipotassium hydrogen phosphate from Sigma Aldrich USA (cat.no.: P3786) in 900 ml water followed by adjusting pH to 7.0 with 4 M hydrochloric acid. Finally, water was added to obtain a final volume of 1 L;
(III) A 0.1 M sodium sulfate solution was prepared by dissolving 14.2 g anhydrous sodium sulfate SL 99.8 % from Lenzing, Austria, in water to a total volume of 1000 ml;
(IV) A 0.1 M pyrocatechol in 0.1 M di-potassium hydrogen phosphate pH 7.0 buffer solution (for PPO detection) was prepared by dissolving 11.01 g pyrocatechol from Sigma Aldrich, USA (cat.no.: C9510) in 100 ml 0.1 M di-potassium hydrogen phosphate pH 7.0;
(V) 20 mg/ml 1-naphtyl acetate in 2-propanol: 80 mg 1-naphtyl acetate from Sigma, USA (Cat.no.: N8505), 4 ml of 2-propanol from VWR BDH Chemicals (Cat.no.: 20922.364);
(VI) 0.1 M tris pH 7.5: 12.1 g Tris VWR Life Science (Cat.no.: 0826), add 900 ml of water adjust pH to 7.5 with 4 % HCl, add water to 1 L;
(VII) Fast Blue BB solution 6 mg/ml in 10 % SDS (freshly made, kept in the dark): 30 mg of Fast Blue BB Salt hemi(zinc chloride) from Sigma, USA (Cat.no.: F3378), 5 ml 10 % SDS;
(VIII) 0.2 mg/ml 1-naphtyl acetate in 0.1 M tris pH 7.5 (freshly made): 30 ml 0.1 M tris pH 7.5, 0.3 ml 20 mg/ml 1-naphtyl acetate in 2-propanol.

### SDS-PAGE electrophoresis reagents

LDS sample buffer, 4X was obtained from Expedeon, USA (Cat.no.: NXB31010)
SDS Run buffer, 20x was obtained from Expedeon, USA (Cat.no.: NXB50500)
Precast 4-20% gradient gels were obtained from Expedeon, USA (Cat.no.: NXG42012K)
Instant Blue Coomassie staining solution was obtained from Expedeon, USA (Cat.no.ISB1L).

### Assays

### Esterase activity

To 3 ml 0.2 mg/ml 1-naphtyl acetate in 0.1 M tris pH 7.5 (fresh made), 0.3 ml potato protein sample is added. The solution is mixed well and incubated for exactly 15 minutes. 0.42 ml of Fast Blue BB solution 6 mg/ml in 10 % SDS is added. The resulting solution is mixed well and incubated for exactly 10 minutes.

A reference sample of 0.3 ml tris buffer is made up (= blind). The samples are analysed at 510 nm (O.D. 510) using a spectrophotometer (BK-UV1800 spectrophotometer, Biobase, China). The 510 nm reading from the blind is subtracted from the 510 nm reading from the potato protein sample. The result is expressed as '*esterase activity signal*' and is used as a relative measure to compare different samples within the same experiment *(i.e.* without transforming into a units/mg expression of activity).

### SDS-PAGE electrophoresis

The samples produced in the examples were analyzed using SDS-PAGE gel electrophoresis to determine the protein composition in each sample. The SDS-PAGE gel electrophoresis was performed using an electrophoresis apparatus and precast 4-20% gradient gels from Expedeon USA (Cat.no.: NXG42012K). The protein samples were mixed with LDS sample buffer and incubated for 10 minutes at 70 °C. The samples were applied to a precast gel and the proteins were allowed to run for one hour at 200 V 90 mA in the SDS Run buffer at non-reduced running conditions. The gel was developed in the staining solution for three hours and the protein bands were evaluated by visual inspection or analyzed by scanning densitometry to quantify the amount of specific proteins in the test solutions.

### Dry matter determination

A Sartorius moisture analyzer (MA37, Sartorius) was used to determine dry matter in a sample by applying 5-10 mL of a sample to the instrument. The sample was dried at 110 °C until constant weight and the remaining dry matter was determined and calculated by the instrument.

### Semi-quantitative determination of polyphenol oxidase (PPO) activity

A sample was mixed with a pyrocathecol solution pH 7.0 at ambient temperature. If the sample contained PPO, the solution changed color from colorless to orange within 15 minutes. The color intensity obtained after 15-minute incubation at room temperature indicates the amount of PPO enzyme activity in the sample. A sample with an unknown amount of enzyme activity may quickly be estimated relative to the activity expressed by a reference sample (e.g. the starting material) and dilutions hereof by visual inspection.

The procedure was as follows. A solution is prepared by combining 1.95 ml 0.1 M potassium phosphate pH 7.0, 1 ml 0.1 M pyrocatechol in 0.1 M potassium phosphate pH 7.0 and 50 µl of a sample. The resulting solution was mixed well and incubated for 15 minutes at ambient temperature. After 15 minutes, the color intensity was scored relative to a reference sample.

### Determination of total glycoalkaloids

A HPLC method for determination of total glycoalkaloids was applied according to V. Alt et al., Optimization of glycoalkaloid analysis for use in industrial potato fruit juice downstreaming, Eng. Life Sci., 2005, 5, pp 562-567. α-solanine (Sigma Aldrich, USA, cat no.: S3757) was used as a reference.

### Test for alkaline colored phenolic compounds

A semi-quantitative test for the content of complex phenolic compounds based on the development of color in alkaline medium was used. A sample with a volume of 0.5 ml was mixed with 3 ml 1 M sodium hydroxide. The color intensity of a set of samples was inspected visually and assigned a relative score as follows:
++++ means very strong color intensity, +++ means strong color intensity, ++ means clearly visible color intensity, + means weak color intensity, (?) means hardly visible color, ND means no visible color.

### True protein determination

True protein concentration was also determined by the method of Kjeldahl using the conversion factor N x 6,25. All samples were initially dialyzed against demineralised water in dialysis tubing cellulose membrane (Sigma-Aldrich, USA, cat. No.: D9652) to remove any free amino acids and low molecular weight peptides.

### Membrane filtration

Membrane filtration test runs were performed using a multipurpose pilot membrane filtration unit (PAN 4010 from Koch Membrane Systems, USA) equipped with the relevant membrane types as indicated in the examples.

### Example 1: Ultrafiltration and diafiltration of potato fruit juice with subsequent isolation of polyphenols and glycoalkaloids

30 L potato fruit juice, produced as described in sections *'Materials'* and *'Assays'* without any pretreatment to clarify the potato fruit juice, was subjected to ultrafiltration followed by diafiltration. The potato fruit juice, having a true protein concentration of 11.2 g/l, had a pH of 6.0 and a conductivity of 13.3 mS/cm. When the potato fruit juice was diluted with water to a 5 mg/ml true protein concentration and centrifuged in a tabletop centrifuge at 4000 G for 30 minutes, there remained a pellet constituting approximately 1 vol/vol% of the potato fruit juice volume. Accordingly, the potato fruit juice comprised a considerable amount of insoluble fibers. O.D. 600 nm of the potato fruit juice was 1.95 (measured as diluted 3X in 50 mM potassium phosphate, at pH 7.0. The resulting absorbance reading was multiplied by 3).

The ultrafiltration and diafiltration permeates obtained were mixed and thereafter subjected to nanofiltration for separation of the polyphenols and glycoalkaloids from salts. Hereafter the polyphenols were adsorbed onto an ion exchanger to separate polyphenols and glycoalkaloids.

### Ultrafiltration

The procedure was as follows: a 3 inch ROMICON PM30, 1.1 mm i.d. (Koch Membrane Systems, USA) hollow fiber membrane unit was employed. This unit has a membrane area of 2.3 m². The cross flow was set at 34 L/min and the TMP (transmembrane pressure) was regulated to be approximately 1.2 bar.

The 30 L potato fruit juice was concentrated using ultrafiltration to a retentate volume of 6 L *(i.e.* the concentration factor was 5X) and the 24 L clear purple/brown permeate (labelled '*first permeate'*) was collected and stored at 2-4 °C until further processing. Using the built-in heat exchanger of the equipment, the temperature of the retentate (labelled '*first retentate')* was kept in the range of 20-25 °C during the entire ultrafiltration procedure.

The flux observed in the beginning of the ultrafiltration process was approximately 35 LMH and thereafter gradually decreased to 15 LMH when the retentate was 5X concentrated. There were no signs of membrane fiber clogging due the high content of insoluble material (insoluble fibers).

### Diafiltration

Following the 5X concentration using ultrafiltration, 6 L, of 3 g/L sodium sulfite was added to the first retentate (Sigma Aldrich USA, cat. No.: 13471), conductivity = 4.1 mS/cm at 18 °C, followed by further ultrafiltration to again reduce the retentate volume to 6 L while the corresponding permeate was collected in a separate vessel labelled *'diafiltrate'.* This procedure was repeated in total 12 times such that in total 72 L diafiltrate was obtained. However, only the first 36 L permeate were collected and stored for the subsequent nanofiltration step. Following the diafiltration with sodium sulfite solution, an additional 3 x 6 L water was added to remove the sodium sulfite from the retentate. The retentate (5.8 L) after diafiltration was labelled '*retentate*'.

During diafiltration, a gradual increase of flux was observed and at the final stages the flux was in the range of 25 LMH when a 6 L diafiltration portion was added. This correlated well with a gradual increase of pH in the retentate for each diafiltration step added which initially had a pH of 5.9 and at the end of diafiltration with sodium sulfite had a pH of 8.3.

Analysis of the first 6 x 6 L *'diafiltrate'* for alkaline colored phenolic compounds showed that the concentration of phenolic compounds was very high in the first permeate fractions and then gradually decreased for each 6 L permeate fraction. The sixth permeate fraction was estimated to contain less than 10 % phenolic compounds than the first permeate. Also, the majority of the total glycoalkaloids were analyzed to be in the first six permeate fractions, although still a significant concentration was also found in the subsequent fractions.

A sample of the *'retentate'* obtained after diafiltration was diluted 20 times in 50 mM potassium phosphate at pH 7.0 and the O.D. 600 of the diluted sample was measured to be 0.47. Thus, the undiluted retentate had an O.D. 600 nm of 20 x 0.47 = 9.4. The undiluted retentate had a pH value of 7.8 and a conductivity of 0.8 mS/cm.

The dry matter concentration of the retentate was measured to be 6.7 %, corresponding to a total protein/insoluble fiber product yield of 5.8 x 67 = 388.6 g. The true protein content was determined by the Kjeldahl method to be 87 % and the total glycoalkaloid content was determined to be 75 mg/kg protein (on a dry matter basis).

SDS-PAGE analysis of potato fruit juice, diafiltration permeates and the retentate after diafiltration was carried out as described in the *'Assays'* section. Results are depicted in Figure 5 (lane 1: potato fruit juice; lanes 2-8: diafiltrates step 1-7; lane 9: retentate after diafiltration). The analysis illustrates that the final protein product in the retentate after diafiltration (lane 9) has substantially the same protein composition as the starting material, *i.e.* potato fruit juice (lane 1). The analysis further illustrates that only very small amounts of potato protein migrates into the diafiltration permeates.

### Nanofiltration and separation of polyphenols and glycoalkaloids by ion exchange

A) The membrane filtration unit was equipped with a spiral wound nanofiltration membrane having an approximate molecular weight cut-off of 600-800 Dalton (Model NFG 2B 2538, Synder Filtration, USA). The '*first permeate'* and the *'diafiltrate'* obtained as described above were mixed to create a total volume of 60 L of a clear purple/brown solution containing the polyphenols and glycoalkaloids isolated from the potato fruit juice during ultrafiltration and diafiltration. The resulting solution had a pH of 6.8 and a conductivity of 10.4 mS/cm. 10 L of the mixed solution was then nanofiltered using a cross-flow of 22 L/min and a TMP of 5 bar. The process temperature as maintained at 20-25 °C. Samples of the permeate and the corresponding retentate were collected when 2 L permeate had been produced, were analyzed for conductivity and were tested for alkaline colored phenolic compounds as described in the *'Assays'* section. The nanofiltration process was conducted until the 10 L mixed solution was concentrated to about 3 L and then stopped.
   The conductivity of the collected nanofiltration permeate and retentate samples were found to be approximately 6.6 mS/cm and 10.9 mS/cm, respectively. This indicates that most of the salts present in the mixture passed the nanofiltration membrane into the permeate. The semi-quantitative test for alkaline colored phenolic compounds indicated that approx. 65 % of the alkaline colored phenolic compounds were retained in the retentate.
(B) The membrane filtration unit was then equipped with a spiral wound nanofiltration membrane having an approximate molecular weight cut-off of 300-500 Dalton (Model NFW 2B 1812F, Synder Filtration, USA), and another portion of 10 L of the mixed solution was then nanofiltered using a cross-flow of 6 L/min and a TMP of 5 bar. The process temperature was maintained at 20-25 °C. Samples of the permeate and the corresponding retentate were collected when 2 L permeate had been produced and were analyzed for conductivity, alkaline colored phenolic compounds and total glycoalkaloids as described in the *'Assays'* section. The nanofiltration process was conducted until the 10 L mixed solution was concentrated to about 2 L and then 2 L water was added with subsequent reconcentration to 2 L retentate. This was repeated once to obtain in total 12 L nanofiltration permeate.
   The conductivity of the collected nanofiltration permeate and retentate samples were found to be approximately 4 mS/cm and 11.2 mS/cm, respectively. This indicates that approximately one third of the salts present in the mixture passed the membrane into the permeate. A significant additional part of the remaining salts were found to pass the membrane during the 2 diafiltration steps with water, which indicates that the polyphenols and total glycoalkaloids can be efficiently separated from the salts in the mixed solution. The semi-quantitative test for alkaline colored phenolic compounds as well as the test for total glycoalkaloids indicated that no detectable amounts of the alkaline colored phenolic compounds nor total glycoalkaloids were passing the membrane which indicated that this nanofiltration membrane retains approximately 100 % of the alkaline colored phenolic compounds and the total glycoalkaloids in the retentate.
   The quality of the obtained permeate enables recycling of the permeate and thereby reuse of the water and salts as diafiltration liquid in the diafiltration step to create the *'diafiltrate'* after 5X concentration of the potato fruit juice as described above. In this way the total water and salt usage of the process according to the invention will be highly reduced.

### Separation of phenolic compounds and total glycoalkaloids by ion exchange

The partly desalted nanofiltration retentate from experiment (B) as described above was applied to a chromatographic column packed with Q Sepharose Fast Flow (Cat no. Q1126, Sigma Aldrich, USA) which was pre-washed and equilibrated with water. The pH of the nanofiltration retentate sample was 6.8. The sample was loaded until the column was nearly completely saturated with the bound phenolic compounds. The run-through (non-bound fraction) from the column was collected and analyzed for the content of alkaline colored phenolic compounds.

The phenolic compounds were found to bind strongly to the Q Sepharose Fast Flow adsorbent, which could be observed by visual inspection of the column in the form of a dark-brown band of bound phenols that appeared at the top and gradually filled the column as more sample was applied. The collected run-through was negative in the test for alkaline colored phenolic compounds, which indicates that substantially all phenolic compounds were bound the adsorbent. While the phenolic compounds can be negatively charged at near-neutral to alkaline pH values, the glycoalkaloids will be neutral or slightly positively charged and do therefore not bind to the adsorbent. Accordingly, they will migrate in the run-through fraction, and are thus separated from the phenolic compounds. The bound phenolic compounds could be efficiently released from the adsorbent with 0.1 M sodium hydroxide.

### Example 2: Ultrafiltration and diafiltration of potato fruit juice with an integrated step for inactivation of enzymatic activity

30 L potato fruit juice, produced as described in sections *'Materials'* and *'Assays'* without any pretreatment, was subjected to ultrafiltration and diafiltration to clarify the potato fruit juice. The potato fruit juice, having a true protein concentration of 11.5 g/l, had a pH of 6.0 and a conductivity of 13.1mS/cm. When the potato fruit juice was diluted with water to a 5 mg/ml true protein concentration and centrifuged in a tabletop centrifuge at 4000 G for 30 min there remained a pellet constituting approximately 1 vol/vol% of the potato fruit juice volume. Accordingly, the potato fruit juice comprised a considerable amount of insoluble fibers. O.D. 600 nm of the potato fruit juice was 2.0 (measured as diluted 3X in 50 mM potassium phosphate, at pH 7.0. The resulting absorbance reading was multiplied by 3).

The retentate obtained after a first diafiltration step was subjected to an enzyme inactivation step, followed by further diafiltration, solubilization and final purification of the resulting protein product.

### Ultrafiltration

The procedure was as follows: a 3 inch ROMICON PM30, 1.1 mm i.d. (Koch Membrane Systems, USA) hollow fiber membrane unit was employed. This unit has a membrane area of 2.3 m². The cross flow was set at 34 L/min and the TMP (transmembrane pressure) was regulated to be approximately 1.2 bar.

The 30 L potato fruit juice was concentrated using ultrafiltration to a retentate volume of 6 L *(i.e.* the concentration factor was 5X) and the 24 L clear purple/brown permeate (labelled '*first permeate*') was collected and stored at 2-4 °C until further processing. Using the built-in heat exchanger of the equipment the temperature of the retentate (labelled '*first retentate')* was kept in the range of 20-25 °C during the entire ultrafiltration procedure.

The flux observed in the beginning of the ultrafiltration process was approximately 35 LMH and thereafter gradually decreased to 15 LMH when the retentate was 5X concentrated. There were no signs of membrane fiber clogging due the high content of insoluble material (insoluble fibers).

### Diafiltration

Following the 5X concentration using ultrafiltration, 6 L, 3 g/L sodium sulfite was added to the first retentate (Sigma Aldrich USA, cat. No.: 13471), conductivity = 4.1 mS/cm at 18 °C, followed by further ultrafiltration to again reduce the retentate volume to 6 L while the corresponding permeate was collected in a separate vessel labelled *'diafiltrate'.* This procedure was repeated in total 6 times such that in total 36 L diafiltrate was obtained. The retentate was labelled '*retentate*'.

A sample of the *'retentate'* obtained after diafiltration was diluted 20 times in 50 mM potassium phosphate at pH 7.0 and the O.D. 600 of the diluted sample was measured to be 0.6. Thus, the undiluted retentate had an O.D. 600 nm of 20 x 0.6 = 12. The undiluted retentate had a pH value of 7.7.

During diafiltration a gradual increase of flux was observed and at the final stages the flux was in the range of 22 LMH when a 6 L diafiltration portion was added. This correlated well with a gradual increase of pH in the retentate for each diafiltration step added which initially had a pH of 5.9 and at the end of this first diafiltration with sodium sulfite had a pH of 7.7.

Analysis of the first 6 x 6 L diafiltration permeate for alkaline colored phenols showed that the concentration of phenols was very high in the first permeate fractions and then gradually decreased for each 6 L permeate fraction. The sixth permeate fraction was estimated to contain less than 10 % of the initial permeates. Also, the majority of the glycoalkaloids were analyzed to be in the first six permeate fractions, although still a significant concentration was also found in the subsequent fractions.

### Inactivation of enzyme activity, further diafiltration, pH adjustment and final diafiltration

A 20 ml sample of the retentate obtained after the first diafiltration step was divided into two aliquots which were adjusted with sulfuric acid to a pH of 4.0 and 3.0, respectively. The two solutions were then again divided into two aliquots which were incubated at room temperature (RT, 20-23 °C) and 40 °C, respectively, for 3 hrs. During the incubation, samples for analysis of esterase activity were withdrawn, neutralized with sodium hydroxide and analyzed for esterase activity as described in the *'Assays'* section.

Figure 6 shows the esterase activity signal (O.D.510 nm) in the different samples as a function of incubation time at pH 4.0 and pH 3.0, respectively. Figure 6 illustrates that the esterase activity in the retentate sample is reduced when incubated at pH 4.0 or 3.0, respectively, at room temperature. At pH 3, the esterase activity signal at 510 nm decreases strongly over time and after 3 hrs., it is reduced with a factor of 24. When the temperature is increased to 40 °C, the inactivation is very effective even after 15 minutes, after which the esterase activity is reduced with a factor of 98. At room temperature and pH 3.0, it is observed that an activity drop of about 85 % is reached after about 1 hour and that this level is rather constant until up to about 2 hours after incubation. From a process robustness perspective, it may be preferable to have ample time to perform the operations without changes of the process outcome and it was therefore chosen to perform the next preparative steps at pH 3.0 and room temperature.

### Preparative inactivation procedure and subsequent solubilization

The retentate after diafiltration was kept in the membrane filtration unit and 6 L, 20 mM sodium sulfate was added (SL 99.8 % from Lenzing, Austria adjusted to pH 3.3 with sulfuric acid, conductivity = 3.8 mS/cm at 20 °C). Thereafter, the pH was adjusted to 3.0 with sulfuric acid while recirculating in the membrane filtration unit using the built-in heat exchanger to keep the temperature in the range of 20-25 °C. Following recirculation for 15 minutes, the retentate was then again concentrated to a volume of 6 L retentate. The permeate was collected and labelled 'acidic permeate' and stored at 2-4 °C. Following the concentration, again 6 L, 20 mM sodium sulfate, pH 3.3, was added to the retentate. This procedure was repeated in total 6 times such that in total 42 L acidic permeate was obtained.

To the retentate was then added 6 L water and the pH was adjusted to pH 8.5 with sodium hydroxide while the retentate was kept in the membrane filtration under recirculation. A total of 2 hours at 20-25 °C was passing from the time the retentate was adjusted to pH 3.0 and until the pH was raised again to pH 8.5. The retentate was then again concentrated to a volume of 6 L retentate. The permeate was collected and labelled '*final permeate'* and stored at 2-4 °C. Following the concentration, 6 L of water was added to the retentate. This procedure was repeated in total 3 times such that in total 24 L final permeate was obtained. The retentate was collected from the membrane filtration unit in a volume of 5.8 L and labelled '*final retentate'.*

The conductivity of the final retentate was 0.8 mS (at 20 °C). The pH was 7.9. The dry matter concentration of the final retentate was 6.5 %, corresponding to a total yield of dry matter of 377 g. The protein purity was determined by Kjeldahl analysis as described in the methods section to be 89 % and the total glycoalkaloid content was determined to be 25 mg/kg protein (on a dry matter basis). Testing for alkaline colored phenolic compounds gave no visible reaction, indicating that practically all phenolic compounds were removed.

When the final retentate was diluted to a true protein concentration equal to the potato fruit juice (11.5 g/L) and was analyzed for esterase activity as described in the *'Assays'* section, it was found that the activity signal was reduced with approximately 85 %. Likewise, when performing the determination of polyphenol oxidase (PPO) activity no visible reaction was observed, indicating that practically all the PPO activity was eliminated.

A sample of the final retentate was diluted 20 times in 50 mM potassium phosphate at pH 7.0 and the O.D. 600 of the diluted sample was measured to be 0.55. Thus, the undiluted final retentate had an O.D. 600 nm of 20 x 0.55 = 11. Accordingly, the final retentate comprised a considerable amount of insoluble fibers.

When diluted to a dry matter concentration of 0.5 % in water and after centrifugation for 30 minutes at 4000 G, there was less than 1 vol/vol% pellet. The dry matter of the supernatant was still close to 0.5 % indicating a more than 90 % solubility of the potato protein product. This is in sharp contrast to prior art reports indicating that low pH treatment of potato fruit juice leads to significant loss of solubility, and thereby functionality.

## Claims

1. A method for separation of (a) potato proteins and insoluble fibers from (b) first salts and phenolic and/or glycoalkaloid compounds in potato fruit juice or a derivative thereof, said method comprising the steps of:
(i) providing a potato fruit juice or a derivative thereof, comprising
• potato proteins; and
• insoluble fibers; and
• one or more first salts; and
• phenolic and/or glycoalkaloid compounds,
wherein the potato fruit juice or the derivative thereof has an absorbance at 600 nm in the range of 0.1 to 50, as determined using the method described in the detailed description and contains insoluble fibers at a concentration in the range of 0.1 to 30 mg/ml, based on the dry weight of the insoluble fiber;
(ii) subjecting the potato fruit juice or the derivative thereof provided in step (i) to a first cross-flow membrane filtration process wherein water and at least a portion of the first salts and at least a portion of the phenolic and/or glycoalkaloid compounds migrate across the membrane into a first permeate and wherein the potato proteins are retained in a first retentate, said first retentate having a true protein concentration in the range of 5 g/L to 200 g/L;
(iii) adding aqueous diafiltration liquid containing one or more salts to the first retentate obtained in step (ii) to form a diluted first retentate having a conductivity of between 0.1 mS/cm and 50 mS/cm and an absorbance at 600 nm of between 0.01 and 50, as determined using the method described in the detailed description, and subjecting said diluted first retentate to a second cross-flow membrane filtration as diafiltration, to create a second permeate being a diafiltrate containing at least a portion of said phenolic and/or glycoalkaloid compounds and salts and a second retentate comprising potato proteins, salts and insoluble fibers.

2. The method according to claim 1, further comprising the step of:
subjecting the first permeate from said first cross-flow membrane filtration process to a third cross-flow membrane filtration process, wherein at least a portion of the salts present in the first permeate migrate across the membrane into a third permeate and wherein the phenolic and/or glycoalkaloid compounds are retained in a third retentate,
and/or, further comprising the step of:
subjecting the second permeate being a diafiltrate from said second cross-flow membrane filtration process to a fourth cross-flow membrane filtration process, whereby at least a portion of the salts present in said second permeate being a diafiltrate migrate across the membrane into a fourth permeate and wherein the phenolic and/or glycoalkaloid compounds are retained in a fourth retentate,
and/or further comprising the step of:
subjecting the second retentate to a fifth membrane filtration process resulting in a fifth retentate, wherein at least a portion of the salts migrate across the membrane into a fifth permeate.

3. The method according to claim 2, wherein aqueous diafiltration liquid is added to the second retentate to form a diluted second retentate before the fifth membrane filtration process, and wherein the fifth membrane filtration process is performed on the diluted second retentate as diafiltration, resulting in a fifth permeate being a diafiltrate and the fifth retentate.

4. The method according to claim 2 or 3, wherein the membrane is a microfiltration membrane and wherein at least a portion of the salts and the proteins soluble in said second retentate migrate across the microfiltration membrane.

5. The method according to any one of claims 2-4, wherein the fourth permeate is used, at least in part, as the source of said aqueous diafiltration liquid containing one or more salts in claim 1, step (iii) and/or
, wherein the aqueous diafiltration liquid containing one or more salts contains one or more second salts that are added to the aqueous diafiltration liquid and that are different from the one or more first salts.

6. The method according to any one of the preceding claims, further comprising the step of adjusting the pH of said potato fruit juice or the derivative thereof to a pH in the range of 4.5 to 8.5, such as in the range of 5.2 to 7.5, preferably in the range of 4.5 to 6.5, 4.8 to 6.5, or 5.0 to 6.5 prior to said first cross-flow membrane filtration process and/or
, further comprising the step of adjusting the conductivity of said potato fruit juice or the derivative thereof to a conductivity in the range of 2-500 mS/cm, such as in the range of 1 to 50 mS/cm, 2 to 40 mS/cm, 3 to 30 mS/cm, 3 to 25 mS/cm, 3 to 20 mS/cm, or 5 to 17 mS/cm prior to said first cross-flow membrane filtration process.

7. The method according to any one of the preceding claims, wherein the potato fruit juice or the derivative thereof is pretreated by centrifugation and/or filtration to remove insoluble material of a particle size larger than 10 micron prior to said first cross-flow membrane filtration process.

8. The method according to any one of claims 2-4, wherein the fifth retentate is further treated with a sixth cross-flow membrane filtration process whereby the protein and insoluble fibers are concentrated in a sixth retentate and the salts migrate through the membrane to create a sixth permeate, preferably wherein the sixth permeate is used as the aqueous diafiltration liquid in claim 3.

9. The method according to claim 5, wherein the one or more second salts are selected from sodium, potassium, ammonium, calcium or magnesium salts of a mineral acid or an organic acid and/or wherein the one or more second salts are selected from salts of monovalent inorganic anions and cations, such as sodium chloride, potassium chloride and ammonium chloride, preferably wherein the one or more second salts are selected from sodium sulfate and potassium sulfate.

10. The method according to any one of the preceding claims, wherein the total volume of water added during the diafiltration step (iii) is in the range of 4 to 20 times the volume of the first retentate, such as 4 to 15 times, 5 to 12 times, 5 to 10 times, 6 to 10 times, 7 to 9 times, or 5 to 9 times the volume of the first retentate.

11. The method according to any one of claims 1-10, wherein the true protein concentration of the first retentate is in the range of 8 g/L to 180 g/L, such as in the range of 10 g/L to 160 g/L, 12 g/L to 150 g/L, 15 g/L to 150 g/L, 20 g/L to 150 g/L, 25 g/L to 150 g/L, 30 g/L to 150 g/L, 20 g/L to 140 g/L, 20 g/L to 130 g/L, 20 g/L to 120 g/L, 20 g/L to 100 g/L, 20 g/L to 80 g/L, 20 g/L to 60 g/L, or 30 g/L to 60 g/L.

12. The method according to any one of the preceding claims, further comprising a germicidal step at a temperature in the range of 2-60 °C, such as in the range of 5-55 °C, 8-52 °C, 10-50 °C, 12-45 °C, 12-25 °C, or 12-18 °C, using high intensity UV light, X-rays, pulsed electric field treatment or high-pressure pasteurization, preferably wherein the germicidal step is performed on the potato fruit juice or the derivative thereof provided in step (i) prior to step (ii) or wherein the germicidal step is performed on the first retentate provided in step (ii) prior to diluting with the aqueous diafiltration liquid in step (iii).

## Patentansprüche

1. Verfahren zur Trennung von (a) Kartoffelproteinen und unlöslichen Fasern von (b) ersten Salzen und phenolischen und/oder Glycoalkaloidverbindungen in Kartoffelfruchtsaft oder einem Derivat davon, wobei das Verfahren die folgenden Schritte umfasst:
(i) Bereitstellen eines Kartoffelfruchtsafts oder eines Derivats davon, umfassend
• Kartoffelproteine; und
• unlösliche Fasern; und
• ein oder mehrere erste Salze; und
• phenolische und/oder Glycoalkaloidverbindungen,
wobei der Kartoffelsaft oder das Derivat davon eine Absorption bei 600 nm im Bereich von 0,1 bis 50 aufweist, wie unter Verwendung des Verfahrens bestimmt, das in der detaillierten Beschreibung beschrieben ist, und unlösliche Fasern in einer Konzentration im Bereich von 0,1 bis 30 mg/ml enthält, basierend auf dem Trockengewicht der unlöslichen Fasern;
(ii) Unterziehen des Kartoffelfruchtsafts oder des Derivats davon, das in Schritt (i) bereitgestellt wurde, eines ersten Querstrom-Membranfiltrationsprozesses, wobei Wasser und mindestens ein Teil der ersten Salze sowie mindestens ein Teil der phenolischen und/oder Glycoalkaloidverbindungen über die Membran in ein erstes Permeat wandern und bei dem die Kartoffelproteine in einem ersten Retentat zurückgehalten werden, wobei das erste Retentat eine tatsächliche Proteinkonzentration im Bereich von 5 g/l bis 200 g/l aufweist;
(iii) Zugabe wässriger Diafiltrationsflüssigkeit, die ein oder mehrere Salze enthält, zu dem ersten Retentat, das in Schritt (ii) erhalten wurde, um ein verdünntes erstes Retentat zu bilden, das eine Leitfähigkeit zwischen 0,1 mS/cm und 50 mS/cm und einer Absorption bei 600 nm zwischen 0,01 und 50 aufweist, wie unter Verwendung des Verfahrens, das in der detaillierten Beschreibung beschrieben ist, bestimmt wurde, und
Unterziehen des verdünnten ersten Retentats einer zweiten Querstrom-Membranfiltration als Diafiltration, um ein zweites Permeat, das ein Diafiltrat ist, das mindestens einen Anteil der phenolischen und/oder Glycoalkaloidverbindungen und Salze enthält, und ein zweites Retentat, das Kartoffelproteine, Salze und unlösliche Fasern enthält, zu erzeugen.

2. Verfahren nach Anspruch 1, ferner umfassend den Schritt des Unterziehens des ersten Permeats aus dem ersten Querstrom-Membranfiltrationsprozess eines dritten Querstrom-Membranfiltrationsprozesses, wobei mindestens ein Teil der vorhandenen Salze im ersten Permeat durch die Membran in ein drittes Permeat wandern und wobei die phenolischen und/oder Glycoalkaloidverbindungen in einem dritten Retentat zurückgehalten werden,
und/oder ferner den Schritt umfassend von:
Unterziehen des zweiten Permeats, das ein Diafiltrat aus dem zweiten Querstrom-Membranfiltrationsprozess ist, eines vierten Querstrom-Membranfiltrationsprozesses, wodurch mindestens ein Teil der Salze, die in dem zweiten Permeat, das ein Diafiltrat ist, vorhanden sind, durch die Membran in ein viertes Permeat wandert und wobei die phenolischen und/oder Glycoalkaloidverbindungen in einem vierten Retentat zurückgehalten werden, und/oder ferner den Schritt umfassend von:
Unterziehen des zweiten Retentats eines fünften Membranfiltrationsprozesses, was in einem fünften Retentat resultiert, wobei mindestens ein Teil der Salze durch die Membran in ein fünftes
Permeat wandert.

3. Verfahren nach Anspruch 2, wobei wässrige Diafiltrationsflüssigkeit zu dem zweiten Retentat zugegeben wird, um ein verdünntes zweites Retentat vor dem fünften Membranfiltrationsprozess zu bilden, und wobei der fünfte Membranfiltrationsprozess an dem verdünnten zweiten Retentat als Diafiltration durchgeführt wird, was in einem fünften Permeat, das ein Diafiltrat ist, und dem fünften Retentat resultiert.

4. Verfahren nach Anspruch 2 oder 3, wobei die Membran eine Mikrofiltrationsmembran ist und wobei zumindest ein Teil der Salze und der Proteine, die in dem zweiten Retentat löslich sind, durch die Mikrofiltrationsmembran wandern.

5. Verfahren nach einem der Ansprüche 2-4, wobei das vierte Permeat zumindest teilweise als die Quelle der wässrigen Diafiltrationsflüssigkeit, die ein oder mehrere Salze enthält, in Anspruch 1, Schritt (iii) verwendet wird und/oder
wobei die wässrige Diafiltrationsflüssigkeit, die ein oder mehrere Salze enthält, ein oder mehrere zweite Salze enthält, die der wässrigen Diafiltrationsflüssigkeit zugegeben werden und die sich von dem einen oder den mehreren ersten Salzen unterscheiden.

6. Verfahren nach einem der vorstehenden Ansprüche, ferner umfassend den Schritt des Einstellens des pH des Kartoffelfruchtsafts oder des Derivats davon auf einen pH im Bereich von 4,5 bis 8,5, wie etwa im Bereich von 5,2 bis 7,5, bevorzugt im Bereich von 4,5 bis 6,5, 4,8 bis 6,5 oder 5,0 bis 6,5, vor dem ersten Querstrom-Membranfiltrationsprozess und/oder
ferner umfassend den Schritt des Einstellens der Leitfähigkeit des Kartoffelfruchtsafts oder des Derivats davon auf eine Leitfähigkeit im Bereich von 2-500 mS/cm, wie etwa im Bereich von 1 bis 50 mS/cm, 2 bis 40 mS/cm, 3 bis 30 mS/cm, 3 bis 25 mS/cm, 3 bis 20 mS/cm oder 5 bis 17 mS/cm, vor dem ersten Querstrom-Membranfiltrationsprozess.

7. Verfahren nach einem der vorstehenden Ansprüche, wobei der Kartoffelfruchtsaft oder das Derivat davon durch Zentrifugation und/oder Filtration vorbehandelt wird, um unlösliches Material mit einer Teilchengröße größer als 10 Mikrometer vor dem ersten Querstrom-Membranfiltrationsprozess zu entfernen.

8. Verfahren nach einem der Ansprüche 2-4, wobei das fünfte Retentat ferner mit einem sechsten Querstrom-Membranfiltrationsprozess behandelt wird, wodurch das Protein und die unlöslichen Fasern in einem sechsten Retentat konzentriert werden und die Salze durch die Membran wandern, um ein sechstes Permeat zu erzeugen, bevorzugt wobei das sechste Permeat als die wässrige Diafiltrationsflüssigkeit in Anspruch 3 verwendet wird.

9. Verfahren nach Anspruch 5, wobei das eine oder die mehreren zweiten Salze ausgewählt sind aus Natrium-, Kalium-, Ammonium-, Calcium- oder Magnesiumsalzen einer Mineralsäure oder einer organischen Säure und/oder wobei das eine oder die mehreren zweiten Salze ausgewählt sind aus Salzen von einwertigen anorganischen Anionen und Kationen, wie Natriumchlorid, Kaliumchlorid und Ammoniumchlorid, bevorzugt wobei das eine oder die mehreren zweiten Salze ausgewählt sind aus Natriumsulfat und Kaliumsulfat.

10. Verfahren nach einem der vorstehenden Ansprüche, wobei das Gesamtvolumen des Wassers, das während des Diafiltrationsschritts (iii) zugegeben wird, im Bereich des 4- bis 20-fachen des Volumens des ersten Retentats ist, wie etwa 4- bis 15-faches, 5- bis 12-faches, 5- bis 10-faches, 6-bis 10-faches, 7- bis 9-Faches oder 5- bis 9-Faches des Volumens des ersten Retentats.

11. Verfahren nach einem der Ansprüche 1-10, wobei die tatsächliche Proteinkonzentration des ersten Retentats im Bereich von 8 g/l bis 180 g/l ist, wie etwa im Bereich von 10 g/l bis 160 g/l, 12 g/l bis 150 g/l, 15 g/l bis 150 g/l, 20 g/l bis 150 g/l, 25 g/l bis 150 g/l, 30 g/L bis 150 g/L, 20 g/L bis 140 g/L, 20 g/L bis 130 g/L, 20 g/L bis 120 g/L, 20 g/L bis 100
g/L, 20 g/L bis 80 g/L, 20 g/L bis 60 g/L, oder 30 g/L bis 60 g/L.

12. Verfahren nach einem der vorstehenden Ansprüche, ferner umfassend einen keimtötenden Schritt bei einer Temperatur im Bereich von 2-60 °C, wie etwa im Bereich von 5-55 °C, 8-52 °C, 10-50 °C, 12-45 °C, 12-25 °C oder 12-18 °C, unter Verwendung von hochintensivem UV-Licht, Röntgenstrahlen, gepulster elektrischer Feldbehandlung oder Hochdruckpasteurisierung, bevorzugt wobei der keimtötende Schritt an dem Kartoffelfruchtsaft oder dem Derivat davon durchgeführt wird, der/das in Schritt (i) bereitgestellt wurde, vor Schritt (ii) oder wobei der keimtötende Schritt an dem ersten Retentat durchgeführt wird, das in Schritt (ii) bereitgestellt wurde, vor der Verdünnung mit der wässrigen Diafiltrationsflüssigkeit in Schritt (iii).

## Revendications

1. Procédé de séparation de (a) des protéines et des fibres insolubles de la pomme de terre (b) des premiers sels et des composés phénoliques et/ou glycoalcaloïdes présents dans le jus de pomme de terre ou un dérivé de celui-ci, ledit procédé comprenant les étapes consistant à :
(i) fournir un jus de pomme de terre ou un dérivé de celui-ci, comprenant
• des protéines de pomme de terre ; et
• des fibres insolubles ; et
• un ou plusieurs premiers sels ; et
• des composés phénoliques et/ou glycoalcaloïdes,
où le jus de pomme de terre ou son dérivé a une absorbance à 600 nm dans la plage de 0,1 à 50, telle que déterminée à l'aide du procédé décrit dans la description détaillée et contient des fibres insolubles à une concentration dans la plage de 0,1 à 30 mg/ml, sur la base du poids sec de la fibre insoluble ;
(ii) soumettre le jus de pomme de terre ou son dérivé pourvu à l'étape (i) à un premier procédé de filtration membranaire à flux croisé où l'eau et au moins une partie des premiers sels et au moins une partie des composés phénoliques et/ou glycoalcaloïdes migrent à travers la membrane dans un premier perméat et où les protéines de pomme de terre sont retenues dans un premier rétentat, ledit premier rétentat ayant une concentration réelle en protéines dans la plage de 5 g/L à 200 g/L ;
(iii) ajouter un liquide de diafiltration aqueux contenant un ou plusieurs sels au premier rétentat obtenu à l'étape (ii) pour former un premier rétentat dilué ayant une conductivité comprise entre 0,1 mS/cm et 50 mS/cm et une absorbance à 600 nm comprise entre 0,01 et 50, telle que déterminée à l'aide du procédé décrit dans la description détaillée, et soumettre ledit premier rétentat dilué à une deuxième filtration membranaire à flux croisé sous forme de diafiltration, pour créer un deuxième perméat qui est un diafiltrat contenant au moins une partie desdits composés phénoliques et/ou glycoalcaloïdes et des sels, et un deuxième rétentat comprenant des protéines de pomme de terre, des sels et des fibres insolubles.

2. Procédé selon la revendication 1, comprenant en outre l'étape consistant à : soumettre le premier perméat dudit premier procédé de filtration membranaire à flux croisé à un troisième procédé de filtration membranaire à flux croisé, où au moins une partie des sels présents dans le premier perméat migrent à travers la membrane dans un troisième perméat et où les composés phénoliques et/ou glycoalcaloïdes sont retenus dans un troisième rétentat,
et/ou, comprenant en outre l'étape consistant à :
soumettre le deuxième perméat qui est un diafiltrat dudit deuxième procédé de filtration membranaire à flux croisé à un quatrième procédé de filtration membranaire à flux croisé, de telle sorte qu'au moins une partie des sels présents dans ledit deuxième perméat qui est un diafiltrat migrent à travers la membrane dans un quatrième perméat et où les composés phénoliques et/ou glycoalcaloïdes sont retenus dans un quatrième rétentat,
et/ou comprenant en outre l'étape consistant à :
soumettre le deuxième rétentat à un cinquième procédé de filtration membranaire résultant en un cinquième rétentat, où au moins une partie des sels migrent à travers la membrane dans un cinquième perméat.

3. Procédé selon la revendication 2, où un liquide de diafiltration aqueux est ajouté au deuxième rétentat pour former un deuxième rétentat dilué avant le cinquième procédé de filtration membranaire, et où le cinquième procédé de filtration membranaire est effectué sur le deuxième rétentat dilué sous forme de diafiltration, résultant en un cinquième perméat qui est un diafiltrat et le cinquième rétentat.

4. Procédé selon la revendication 2 ou 3, où la membrane est une membrane de microfiltration et où au moins une partie des sels et des protéines solubles dans ledit deuxième rétentat migrent à travers la membrane de microfiltration.

5. Procédé selon l'une quelconque des revendications 2 à 4, où le quatrième perméat est utilisé, au moins en partie, comme source dudit liquide de diafiltration aqueux contenant un ou plusieurs sels dans la revendication 1, étape (iii) et/ou,
où le liquide de diafiltration aqueux contenant un ou plusieurs sels contient un ou plusieurs deuxièmes sels qui sont ajoutés au liquide de diafiltration aqueux et qui sont différents de l'un ou plusieurs premiers sels.

6. Procédé selon l'une quelconque des revendications précédentes, comprenant en outre l'étape d'ajustement du pH dudit jus de pomme de terre ou du dérivé de celui-ci à un pH dans la plage de 4,5 à 8,5, tel que dans la plage de 5,2 à 7,5, de préférence dans les plages de 4,5 à 6,5, 4,8 à 6,5 ou de 5,0 à 6,5 avant ledit premier procédé de filtration membranaire à flux croisé et/ou,
comprenant en outre l'étape d'ajustement de la conductivité dudit jus de pomme de terre ou du dérivé de celui-ci à une conductivité dans la plage de 2 à 500 mS/cm, tel que dans les plages de 1 à 50 mS/cm, 2 à 40 mS/cm, 3 à 30 mS/cm, 3 à 25 mS/cm, 3 à 20 mS/cm ou de 5 à 17 mS/cm avant ledit premier procédé de filtration membranaire à flux croisé.

7. Procédé selon l'une quelconque des revendications précédentes, où le jus de pomme de terre ou du dérivé de celui-ci est prétraité par centrifugation et/ou filtration afin d'éliminer les matières insolubles dont la taille des particules est supérieure à 10 microns avant ledit premier procédé de filtration membranaire à flux croisé.

8. Procédé selon l'une quelconque des revendications 2 à 4, où le cinquième rétentat est en outre traité avec un sixième procédé de filtration membranaire à flux croisé de telle sorte que la protéine et les fibres insolubles soient concentrées dans un sixième rétentat et les sels migrent à travers la membrane pour créer un sixième perméat, de préférence où le sixième perméat est utilisé sous forme de liquide de diafiltration aqueux dans la revendication 3.

9. Procédé selon la revendication 5, où l'un ou plusieurs deuxièmes sels sont sélectionnés parmi les sels de sodium, de potassium, d'ammonium, de calcium ou de magnésium d'un acide minéral ou d'un acide organique et/ou où l'un ou plusieurs deuxièmes sels sont sélectionnés parmi les sels d'anions et de cations inorganiques monovalents, tels que le chlorure de sodium, le chlorure de potassium et le chlorure d'ammonium, de préférence où l'un ou plusieurs deuxièmes sels sont sélectionnés parmi le sulfate de sodium et le sulfate de potassium.

10. Procédé selon l'une quelconque des revendications précédentes, où le volume total d'eau ajouté pendant l'étape de diafiltration (iii) est dans la plage de 4 à 20 fois le volume du premier rétentat, par exemple de 4 à 15 fois, 5 à 12 fois, 5 à 10 fois, 6 à 10 fois, 7 à 9 fois ou de 5 à 9 fois le volume du premier rétentat.

11. Procédé selon l'une quelconque des revendications 1 à 10, où la concentration réelle en protéines du premier rétentat est dans la plage de 8 g/L à 180 g/L, par exemple dans les plages de 10 g/L à 160 g/L, 12 g/L à 150 g/L, 15 g/L à 150 g/L, 20 g/L à 150 g/L, 25 g/L à 150 g/L, 30g/L à 150 g/L, 20 g/L à 140 g/L, 20 g/L à 130 g/L, 20 g/L à 120 g/L, 20 g/L à 100 g/L, 20 g/L à 80 g/L, 20 g/L à 60 g/L, ou 30 g/L à 60 g/L.

12. Procédé selon l'une quelconque des revendications précédentes, comprenant en outre une étape germicide à une température dans la plage de 2 à 60 °C, par exemple dans les plages de 5 à 55 °C, 8 à 52 °C, 10 à 50 °C, 12 à 45 °C, 12 à 25 °C ou de 12 à 18 °C, en utilisant un rayonnement UV à haute intensité, des rayons X, un traitement par champ électrique pulsé ou une pasteurisation à haute pression, de préférence où l'étape germicide est effectuée sur le jus de pomme de terre ou le dérivé de celui-ci pourvu à l'étape (i) avant l'étape (ii) ou dans laquelle l'étape germicide est effectuée sur le premier rétentat pourvu à l'étape (ii) avant la dilution avec le liquide de diafiltration aqueux à l'étape (iii).
